# EUROPEAN PATENT SPECIFICATION

(11) **EP 2 361 309 B1**
(45) Date of publication and mention of the grant of the patent: **27.07.2016**
(21) Application number: 09760254.4
(22) Date of filing: 12.11.2009
(51) Int. Cl.: A61K 48/00, C12N 9/00, C12N 9/78, C12N 9/88, C12N 15/867, C12N 9/02

(54) **METHOD**
VERFAHREN
PROCÉDÉ

(30) Priority: 11.11.2008 GB 0820628; 01.10.2009 GB 0917241
(43) Date of publication of application: 31.08.2011
(73) Proprietor: Oxford BioMedica (UK) Limited, Oxford Science Park Oxford OX4 4GA (GB)
(72) Inventor: KINGSMAN, Susan, Oxford OX4 4GA (GB); RALPH, Scott, Oxford OX4 4GA (GB); MITROPHANOUS, Kyriacos, Oxford OX4 4GA (GB); PALFI, Stéphane, Oxford OX4 4GA (GB); JARRAYA, Béchir, Oxford OX4 4GA (GB)
(74) Representative: D Young & Co LLP
(86) International application number: PCT/GB2009/002645
(87) International publication number: WO 2010/055290

(56) References cited:
- WO-A2-02/29065
- KIRIK DENIZ ET AL: "Reversal of motor impairments in parkinsonian rats by continuous intrastriatal delivery of L-dopa using rAAV-mediated gene transfer" PROCEEDINGS OF THE NATIONAL ACADEMY OF SCIENCES OF THE UNITED STATES OF AMERICA, vol. 99, no. 7, 2 April 2002 (2002-04-02), pages 4708-4713, XP002572031 ISSN: 0027-8424
- GALVAN A ET AL: "Pathophysiology of Parkinsonism" CLINICAL NEUROPHYSIOLOGY, ELSEVIER SCIENCE, IE, vol. 119, no. 7, 1 July 2008 (2008-07-01), pages 1459-1474, XP022715348 ISSN: 1388-2457 [retrieved on 2008-05-07]
- HEIMER GALI ET AL: "Dopamine replacement therapy reverses abnormal synchronization of pallidal neurons in the 1-methyl-4-phenyl-1,2,3,6-tetrahydropyridi ne primate model of parkinsonism." THE JOURNAL OF NEUROSCIENCE : THE OFFICIAL JOURNAL OF THE SOCIETY FOR NEUROSCIENCE 15 SEP 2002, vol. 22, no. 18, 15 September 2002 (2002-09-15), pages 7850-7855, XP002572032 ISSN: 1529-2401
- CARLSSON THOMAS ET AL: "Reversal of dyskinesias in an animal model of Parkinson's disease by continuous L-DOPA delivery using rAAV vectors", BRAIN, OXFORD UNIVERSITY PRESS, OXFORD, GB, vol. 128, no. Part 3, 1 March 2005 (2005-03-01), pages 559-569, XP002455717, ISSN: 0006-8950, DOI: 10.1093/BRAIN/AWH374
- WONG LIANG-FONG ET AL: "Lentivirus-mediated gene transfer to the central nervous system: Therapeutic and research applications", HUMAN GENE THERAPY, MARY ANN LIEBERT, NEW YORK ,NY, US, vol. 17, no. 1, 1 January 2006 (2006-01-01), pages 1-9, XP002471749, ISSN: 1043-0342, DOI: 10.1089/HUM.2006.17.1
- SHIN-ICHI MURAMATSU ET AL: 'Behavioral Recovery in a Primate Model of Parkinson's Disease by Triple Transduction of Striatal Cells with Adeno-Associated Viral Vectors Expressing Dopamine-Synthesizing Enzymes' HUMAN GENE THERAPY vol. 13, no. 3, 10 February 2002, pages 345 - 354, XP055072278 DOI: 10.1089/10430340252792486 ISSN: 1043-0342
- ANONYMOUS: 'Abstracts of the Proceedings of the XVIIIth Congress of the European Society for Stereotactic and Functional Neurosurgery (ESSFN); 5â 8 October 2008, Rimini, Italy' ACTA NEUROCHIRURGICA ; THE EUROPEAN JOURNAL OF NEUROSURGERY, SPRINGER-VERLAG, VI vol. 150, no. 9, 16 September 2008, pages 933 - 1012, XP019660267 DOI: 10.1007/S00701-008-0030-6 ISSN: 0942-0940

## Description

### FIELD OF THE INVENTION

The present invention relates to dopamine replacement gene therapy for use in the prevention and/or treatment of Parkinson's disease.

### BACKGROUND TO THE INVENTION

Dopaminergic replacement is believed to be the most effective therapeutic strategy for Parkinson's disease (PD) currently in use. Initially, patients with PD experience excellent benefits from pharmacological treatment with the dopamine precursor L-Dopa. However, with chronic L-Dopa intake, most PD patients display fluctuations in motor response to the drug, and develop involuntary abnormal movements called dyskinesias. In many cases, patients cycle between ON-drug periods, which are complicated by disabling dyskinesias, and OFF-drug periods in which the patients are akinetic. It is thought that dyskinesias and motor fluctuations are at least partially caused by the intermittent oral intake of L-Dopa and subsequent pulsatile stimulation of striatal dopamine receptors. This concept suggests that continuous delivery of dopamine may prevent dyskinesias, by restoring dopaminergic tone in the striatum.

One way of achieving continuous dopamine production in the striatum is to use cell grafting of foetal tissue or stem cells. However grafting of stem cells into the substantia nigra has met with limited success (Lindvall O. & Bjorklund A. (2004) NeuroRx 1: 382-393; Kordower J.H. et al (2008) Nature Med 14: 504-506; Li J-Y et al (2008) Nature Med 14: 501-503; Braak H. & Del Tredici K. (2008) Nature Med 14: 483-485). There is therefore a need for a therapy for Parkinson's disease which restores or maintains dopaminergic tone in the striatum, but which avoids the problems associated with foetal tissue/stem cell approaches.

The second type of complication associated with oral L-Dopa treatment is cognitive impairment. PD is characterized primarily by dopamine depletion in the dorsal striatum ('motor' striatum), whereas dopamine function in the ventral striatum ('cognitive' striatum) and also the prefrontal cortex is relatively intact or even upregulated. Because pharmacological L-Dopa treatment stimulates all the brain dopaminergic systems, it critically 'over-doses' the intact ventral striatum and cortex, and impairs associated cognitive functions. This mechanism might also account for other side effects of systemic L-Dopa treatment, such as psychotic symptoms or pathological gambling. Thus, a major challenge in PD is to restore both tonic and local striatal dopamine levels to the dorsal striatum.

Jarraya et al. describe the functional efficacy of Lenti-TH-AADC-CH1 striatal administration in different non human primate models of nigrostriatal degeneration (ACTA NEUROCHIRURGICA; The European Journal of Neurosurgery; vol: 150(9):933-1012).

The present inventors have used a dopamine replacement gene therapy strategy and demonstrated its biochemical and functional efficacy in a non-human primate model of PD.

Surprisingly, the *in vivo* primate data showed many unexpected advantages of dopamine replacement by gene therapy, which could not have been predicted from the *in vitro* or rodent model data, as set out below:
(i) dopamine replacement gene therapy restores both tonic and local striatal dopamine levels to the dorsal striatum. Unlike L-dopa treatment, dopamine replacement gene therapy increases dopamine in the dorsal ("motor") striatum, without over-dosing the ventral ("cognitive") striatum or the pre-frontal cortex. This avoids the impairment of cognitive functions associated with the ventral stratum.
(ii) dopamine replacement gene therapy prevents dyskinesias by restoring dopaminergic tone in the striatum, while avoiding the problems associated with stem cell grafting. The present inventors have found that one of the key factors in the success of any dopamine replacement strategy is the absence of the dopamine active transporter (DAT) which are expressed on the nigral dopaminergic terminals innervating the striatum. Stem cell/foetal tissue strategies usually involve grafting of dopaminergic cells into the substantia nigra of the patient. Levels of DAT are relatively high in the grafted tissue. Without wishing to be bound by theory, the present inventors predict that the presence of DAT sequesters extracellular dopamine produced by the grafted cells, thereby reducing the capacity for the therapy to restore dopaminergic tone. In the dopamine replacement gene therapy method used in the present invention, the vector delivers the genes involved in dopamine synthesis to the striatum. Since the number of dopaminergic terminals and hence DAT levels in the striatum are reduced in patients with Parkinson's disease dopamine production is thus more effective;
(iii) the dopamine replacement gene therapy strategy used in the present invention corrects motor dysfunction associated with Parkinson's disease. The therapy was found to normalise the abnormal over-activity of output nuclei such as the internal globus pallidus (GPi). Analysis of the pattern of neuronal firing in the GPi revealed that the dopamine replacement gene therapy strategy used in the present invention decreased the number of spikes per burst and the number of burst events to levels similar to those observed in non-PD controls. The dopamine replacement gene therapy strategy used in the present invention also normalised the metabolic activity of the subthalamic nucleus (STN). Neuronal hyperactivity in the STN is a pathophysiological feature of PD which causes an increase in metabolic activity detectable in PD subjects;
(iv) the dopamine replacement gene therapy strategy used in the present invention causes sub-physiological levels of dopamine to be produced in the striatum, however, this was sufficient to restore dopaminergic tone, correct motor deficits, prevent drug-induced dyskinesias and restore the normal physiology of key basal ganglia output nuclei; and
v) the dopamine gene replacement strategy used in the present invention can ameliorate dyskinesias associated with oral L-dopa administration. This means that the gene-based approach may both provide therapeutic efficacy in a PD patient that has already developed dyskinesias from long term L-Dopa treatment and may reverse the physiological mechanism of dyskinesias and thus prevent subsequent occurrences following L-Dopa therapy.

### SUMMARY OF ASPECTS OF THE PRESENT INVENTION

The present invention provides a lentiviral vector system which comprises a single vector comprising nucleic acid sequences which encode tyrosine hydroxylase (TH), amino acid DOPA decarboxylase (AADC) and GTP-cyclohydrolasel (CH1) for use in treating and/or preventing dyskinesias associated with oral L-Dopa administration in a Parkinson's disease subject.

The vector system may have one or more of the following features:
(i) where the vector system comprises a tricistronic cassette, the order of the genes in the tricistronic cassette is TH-AADC-CH1
(ii) at least one of the ATG potential start codons in gag is changed to ATTG;
(iii) a Neo expression cassette is inserted downstream of gag; and
(iv) where the vector system comprises a Tricistronic cassette, a WPRE is inserted at the 3' end of the Tricistronic cassette to enhance expression

### FURTHER ASPECTS OF THE PRESENT DISCLOSURE

The present disclosure provides a method for treating and/or preventing Parkinson's disease in a subject without causing cognitive impairment by using dopamine replacement gene therapy to maintain or restore constant physiological dopaminergic tone in both the dorsal and ventral striatum of the subject.

The present disclosure provides a method for normalising neuronal electrical activity in basal ganglia and/or subthalamic nucleus in a Parkinson's disease subject by administration of a vector system for dopamine replacement gene therapy to the subject.

Administration of the vector system may reduce, normalise or prevent a PD-associated increase in the number of spikes per burst and/or the number of burst events in the pattern of neuronal firing in the GPi.

The present disclosure provides a method for treating and/or preventing dyskinesias associated with oral L-dopa administration in a Parkinson's disease subject by administration of a vector system for dopamine replacement gene therapy to the subject.

The present disclosure provides a method for reducing the daily dose of L-Dopa required to maintain locomotor activity.

The present disclosure also provides a vector system for:
(i) increasing the efficacy of dopamine replacement gene therapy for treating and/or preventing Parkinson's disease in a subject by avoiding sequestration of dopamine by the dopamine transporter (DAT);
(ii) treating and/or preventing Parkinson's disease in a subject without causing cognitive impairment by using dopamine replacement gene therapy to maintain or restore constant physiological dopaminergic tone in both the dorsal and ventral striatum of the subject.
(iii) normalising neuronal electrical activity in basal ganglia and/or subthalamic nucleus in a Parkinson's disease subject.
(iv) treating and/or preventing dyskinesias associated with oral L-dopa administration in a Parkinson's disease subject by administration of a vector system for dopamine replacement gene therapy to the subject.

The present disclosure also provides the use of a vector system as defined herein in the manufacture of a pharmaceutical composition for:
(i) increasing the efficacy of dopamine replacement gene therapy for treating and/or preventing Parkinson's disease in a subject by avoiding sequestration of extracellular dopamine by the dopamine transporter (DAT);
(ii) treating and/or preventing Parkinson's disease in a subject without causing cognitive impairment by using dopamine replacement gene therapy to maintain or restore constant physiological dopaminergic tone in both the dorsal and ventral striatum of the subject.
(iii) normalising neuronal electrical activity in basal ganglia and/or subthalamic nucleus in a Parkinson's disease subject.
(iv) treating and/or preventing dyskinesias associated with oral L-dopa administration in a Parkinson's disease subject by administration of a vector system for dopamine replacement gene therapy to the subject.

The present disclosure also provides a method for increasing the efficacy of dopamine replacement gene therapy for treating and/or preventing Parkinson's disease in a subject which comprises the step of avoiding sequestration of extracellular dopamine by the dopamine transporter (DAT).

In the dopamine replacement gene therapy method, for example, sequestration of extracellular dopamine is avoided by administration of the vector system to a tissue which lacks significant DAT expression, such as the Parkinsonian striatum.

Alternatively or in addition, sequestration of dopamine may be avoided by inhibition of DAT expression and/or activity in the subject.

In connection with the above-mentioned aspects, the vector system used for dopamine replacement gene therapy may comprise nucleic acid sequences which encode TH, AADC and CH1, and have one or more of the following features:
(i) at least one of the nucleic acid sequences lack an N-terminal tag;
(ii) at least one of the nucleic acid sequences is codon optimised;
(iii) where the vector system comprises a tricistronic cassette, the order of the genes in the tricistronic cassette is TH-AADC-CH1
(iv) at least one of the ATG potential start codons in gag is changed to ATTG;
(v) a Neo expression cassette is inserted downstream of gag; and
(vi) where the vector system comprises a tricistronic cassette, a WPRE is inserted at the 3' end of the Tricistronic cassette to enhance expression.

The vector system used for dopamine replacement gene therapy may comprise a single vector having nucleic acid sequences which encode TH, AADC and CH1.

The vector system may, for example, be a lentiviral or adeno-associated viral vector system.

### DESCRIPTION OF THE FIGURES

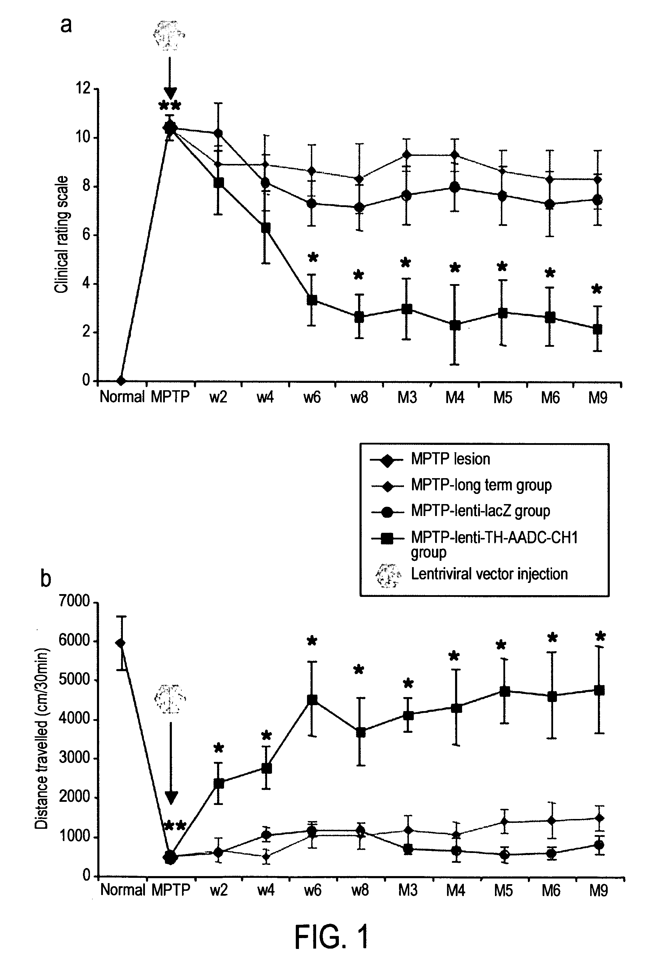

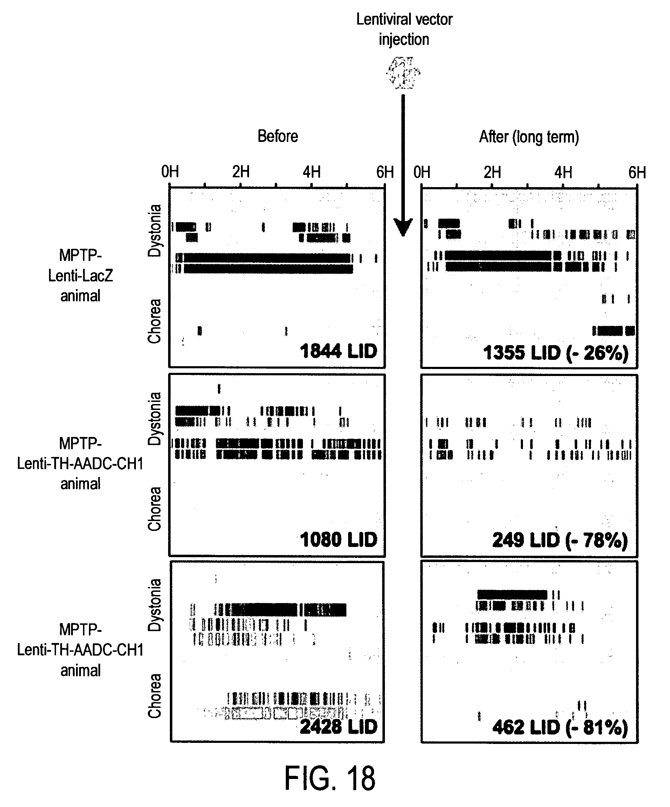

**Figure 1** **: Lenti TH-AADC-CH1 corrects Parkinsonism.**
   Macaques treated with MPTP (black line, n=18) were significantly impaired compared to their control pre-MPTP state, displaying a severe Parkinsonism (a, b). As early as two weeks post lentiviral injection, the animals that received Lenti-TH-AADC-CH1 encoding TH, AADC and CH1 (blue lines, n=6 until w8 then n=3 until M9) had a significant improvement in akinesia (b) compared with the MPTP animals that received Lenti-lacZ (red lines, n=6 until w8 then n=3 until M9) or no viral injection (grey lines, n=6 until w8 then n=3 until M9). Behavioural benefit was sustained up to 9 months post Lenti-TH-AADC-CH1 injection compared to MPTP and MPTP-Lenti-lacZ control animals (a,b). One MPTP-Lenti-TH-AADC-CH1 animal was followed for 30 months after lentiviral injection, and showed stable motor correction. (w, week after gene transfer; M, Month after gene transfer) **p< 0.01 relative to Normal pre-MPTP lesion state, *p< 0.05 relative to MPTP-Lenti-lacZ and to MPTP-long term animals. All data are expressed as mean ± s.e.m.

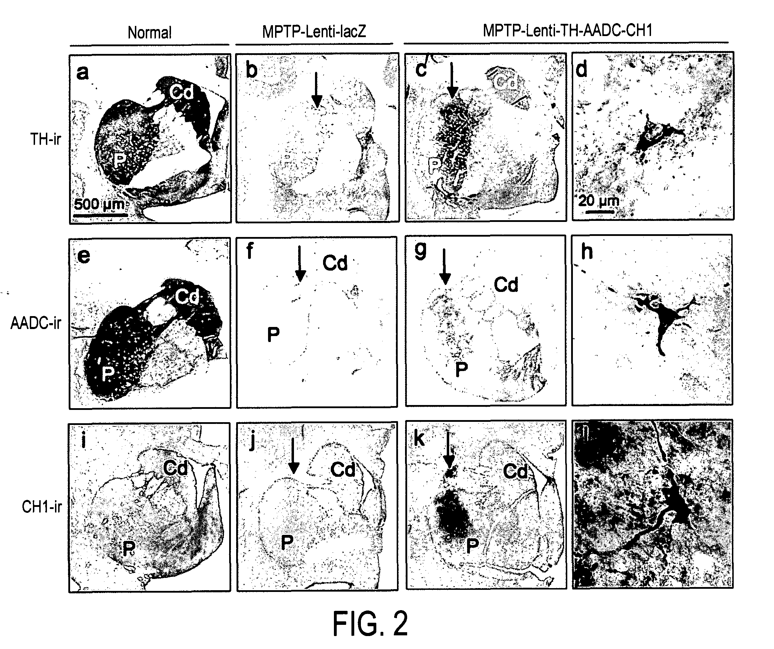

**Figure 2** **: Expression of transgenes after striatal delivery of Lenti-TH-AADC-CH1 viral vector.**
   At low magnification (A-C, E-G, I-K), TH, AADC and CH1 immunoreactivities were highly reduced, especially in the dorsal aspect of the striatum of MPTP-Lenti-lacZ animals (B, F, J) compared to normal unlesioned animals (A, E, I). In contrast, marked increases in TH (C), AADC (G) and CH1 (K) immunoreactivity was seen at the vicinity of the needle track in the commissural and post commissural putamen of MPTP-Lenti-TH-AADC-CH1 infused animals. Higher magnification photomicrographs of Lenti-TH-AADC-CH1 infused areas shows immunoreactive fibers throughout the putaminal neuropile and neurons positive for TH, AADC and CH1 (D, H, L). Arrows show needle tracks. The striatum has been delineated in figure F,J,K. (P, putamen; Cd, caudate nucleus; Bar scale in A applies to all figures except D,H,L; Bar scale in D applies to figures D, H and L).

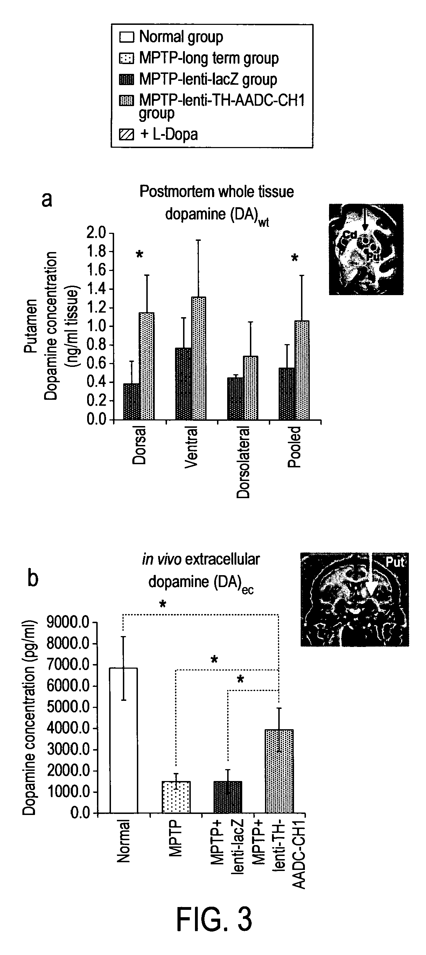

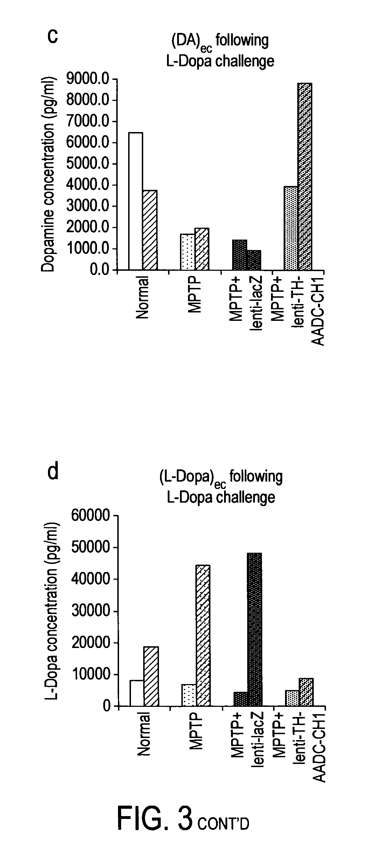

**Figure 3****: Lenti-TH-AADC-CH1 restores striatal dopaminergic tone.**
   **a *postmortem* whole tissue dopamine [DA]_{wt}** Diagrammatic representation of dopamine concentrations measured in punches taken from putamen. Samples were taken from Lenti-lacZ injected (red bars) and Lenti-TH-AADC-CH1 injected (blue bars) MPTP macaques post-mortem. * represents a significant increase in dopamine levels compared with MPTP-Lenti-lacZ controls (p<0.05, n=3).
   **b *in vivo* extracellular dopamine [DA]_{ec}** Extracellular dopamine levels in normal (unlesioned, no gene transfer, white bar) and in MPTP primates that received Lenti-TH-AADC-CH1 (MPTP-Lenti-TH-AADC-CH1, blue bar), Lenti-lacZ (MPTP-Lenti-lacZ, red bar) or no treatment (MPTP-long term, grey bar). Microdialysis probes were placed in the post-commissural putamen for each animal as demonstrated by *in vivo* T2*MRI imaging following the microdialysis procedure. Baseline dopamine levels were reduced to 26% of normal dopamine levels in the MPTP animals indicating a severe dopamine depletion in this animal. Lenti-TH-AADC-CH1, but not Lenti-lacZ, significantly increased striatal extracellular dopamine levels [DA]_{ec} in the treated animals (respectively 60% and 23% of Normal primates, Post-hoc MW p<0.05).
   **c** Following L-Dopa challenge, extracellular dopamine levels were increased 2.25-fold in the MPTP-Lenti-TH-AADC-CH1 animal compared to 1.17-fold with MPTP-long term animal, suggesting that AADC gene transfer may allow a synergy between L-Dopa and Lenti-TH-AADC-CH1.
   **d** Following L-Dopa challenge, extracellular L-Dopa levels in the striatum were increased only in the MPTP-*long term* and MPTP-Lenti-LacZ group following L-Dopa injection suggesting that most of the injected L-Dopa was converted into DA in normal and MPTP- Lenti-TH-AADC-CH1 animals.

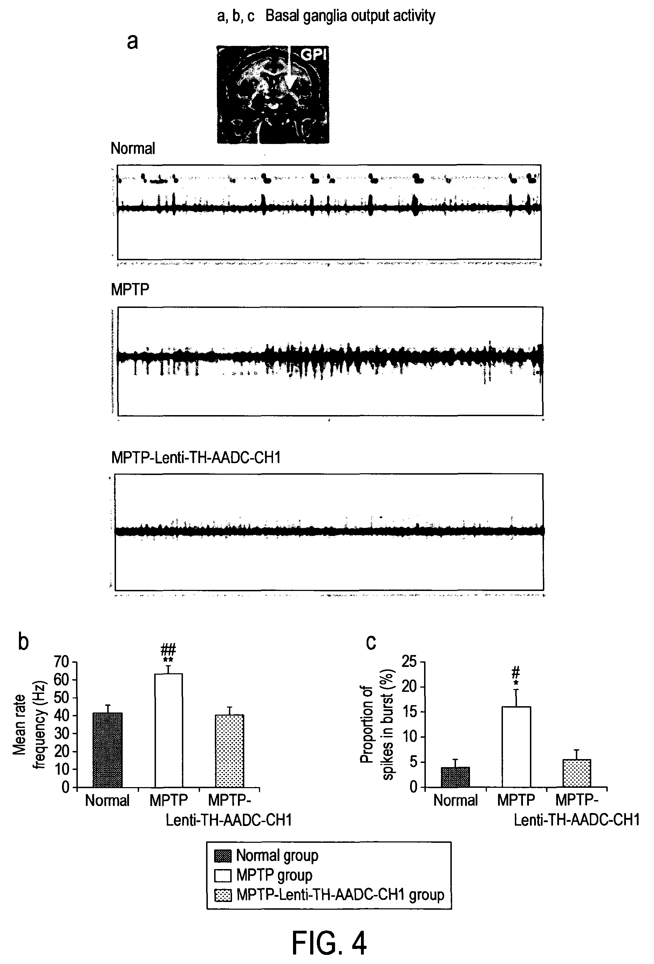

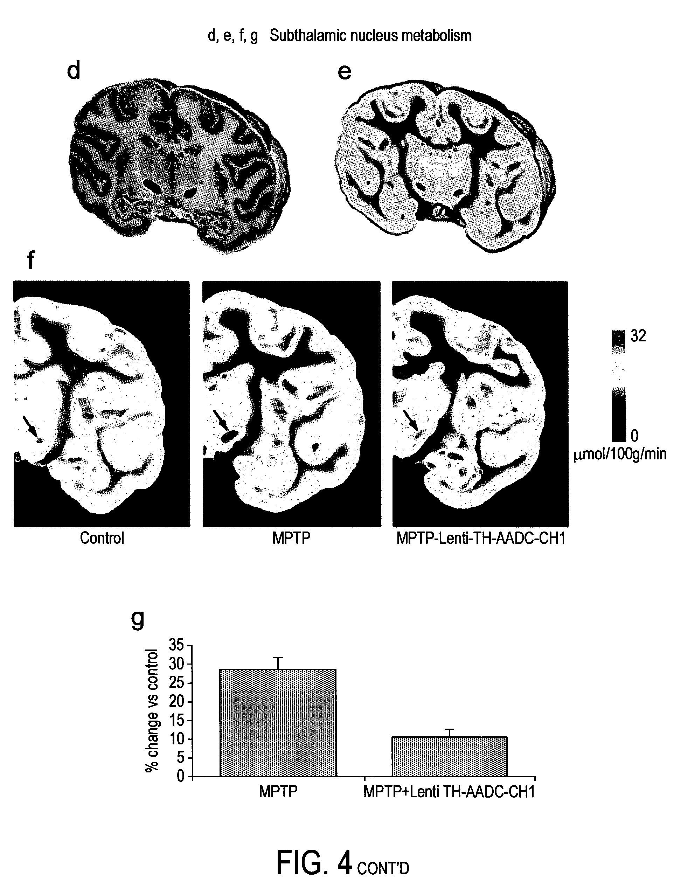

**Figure 4****: Lenti-TH-AADC-CH1 restores normal basal ganglia functioning**
   **a,b,c** Unitary recording (>20 GPi neurons) in normal, MPTP and MPTP-Lenti-TH-AADC-CH1 animals.
   **a** Illustration of 2-second basal rest single neuronal activity within basal ganglia output (GPi). Note a significant increase in the mean firing rate of GPi neurons, and higher burst activity, in drug naïve untreated MPTP macaques compared to controls. Lentiviral dopamine gene therapy significantly reduced abnormal high firing rates in MPTP GPi neurons, restoring normal firing rate values in this structure **b** and normal burst rate **c**.
      *p<0.05, ** p<0.01 relative to normal unlesioned animals
      # p<0.05, ## p<0.01 relative to MPTP-Lenti-TH-AADC-CH1 animals
   **d,e,f,g** Normalization of the metabolic activity within the subthalamic nucleus (STN) after injection of Lenti-TH-AAADC-CH1 into the motor striatum of a MPTP treated primate, as evidenced by 3D [¹⁴C]2-deoxyglucose (2-DG) imaging.
   **d** The right and left STN were manually segmented on the Nissl stained brain sections after 3D reconstruction (10 to 13 sections per STN). These two volumes of interest (VOIs) were directly mapped onto the corresponding autoradiographic co-registered volume **e**.
   **f** Individual left hemibrains autoradiographic images taken at the same level of the STN from one control primate, one MPTP treated primate and one MPTP treated primate who was injected with Lenti-TH-AADC-CH1 52 weeks before the imaging study. Signal intensities are colour-coded according to the same quantitative scale of glucose use (right). **g** Note that the hypermetabolic activity of STN, observed within the MPTP treated primate (+28.6 % of normal control), was normalized by a single dose of Lenti-TH-AADC-CH1 (+ 10.9 % of normal control).

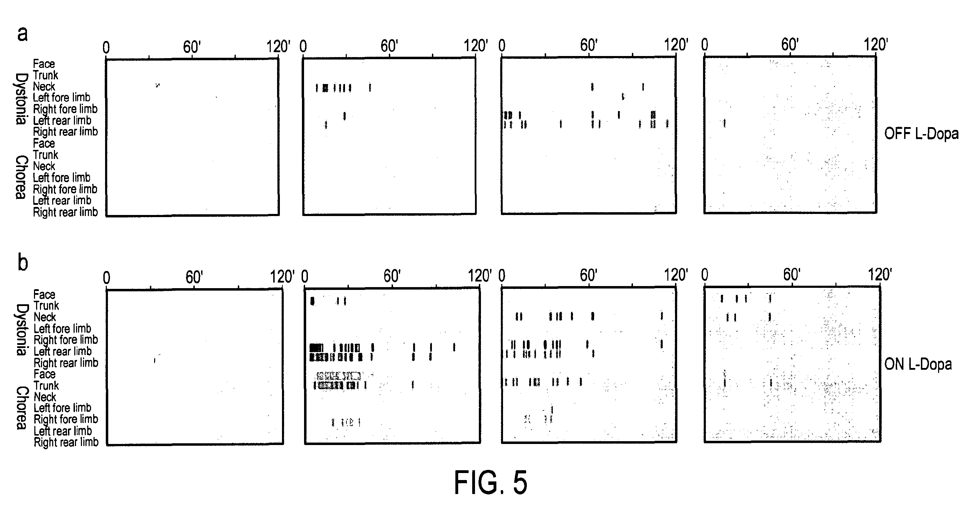

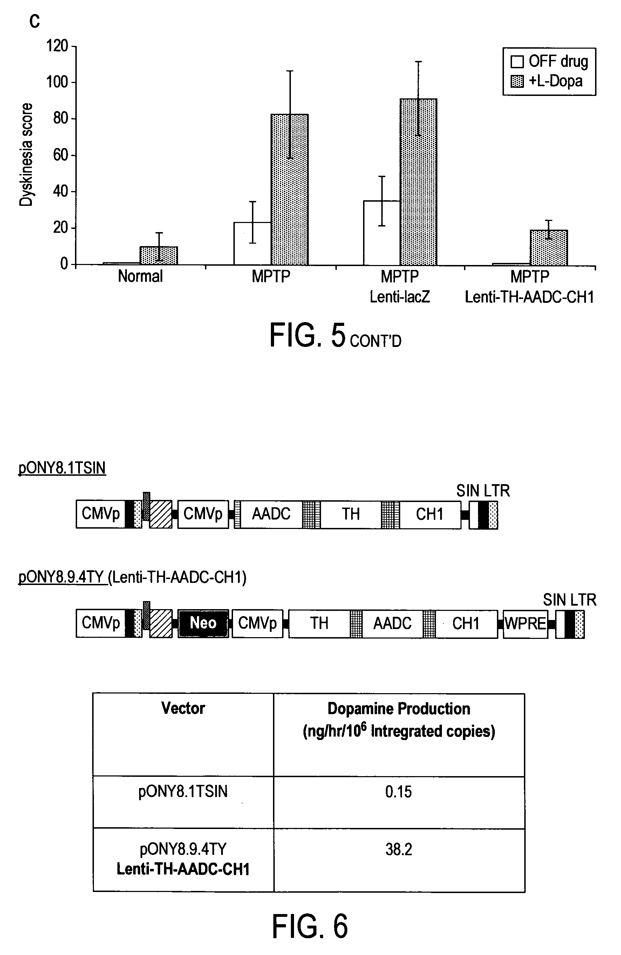

**Figure 5****: Lenti-TH-AADC-CH1 prevents dyskinesia**
   **a Lenti-TH-AADC-CH1 induces no OFF drug dyskinesia.** Although Lenti-TH-AADC-CH1 mediated dopamine corrected motor behaviour to the same level as that obtained by systemic L-Dopa (Figure S12), it did not induce dyskinesia at long term (9 months).
   b Lenti-TH-AADC-CH1 prevents L-Dopa induced dyskinesia. Using pharmacological manipulation of the dopaminergic system, we studied the interaction between endogenous levels of dopamine and exogenous dopamine (L-Dopa). Acute systemic administration of L-Dopa induced dyskinetic movements such as chorea and dystonia, in drug naive MPTP and MPTP-Lenti-lacZ animals. By contrast, normal unlesioned animals, as MPTP Lenti-TH-AADC-CH1 animals, did not show any evidence of dyskinetic movements.
**Figure 6****: Lentiviral dopamine production *in vitro*** pONY8.1TSIN was the vector used in the previous rat study (Azzouz et al (2002) J. Neurosci. 22: 10302-10312). The new vector pONY8.9.4TY (Lenti-TH-AADC-CH1) used in the present study differs from the above in that it has codon optimized genes, no N-terminal peptide tags and the order of genes has been changed but the IRES sequences (blocked boxes) used remain the same. In addition the vector backbone contains a 5' neo gene and a 3' WPRE. In addition all the ATGs in the gag region were mutated to ATTG (box with thick diagonal lines). These changes led to an increase in dopamine production of 2 logs as compared *in vitro* in HEK293T cells.

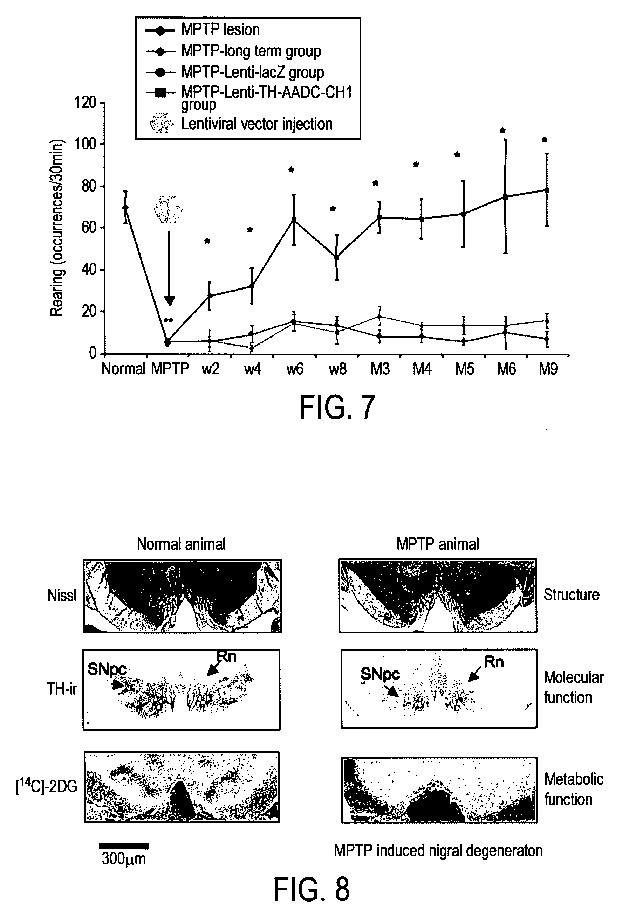

**Figure 7****: Rearing activity**
   Macaques treated with MPTP (black line, n=18) were significantly impaired compared to their control pre-MPTP state, displaying a severe Parkinsonism (a, b). As early as two weeks post lentiviral injection, the animals that received Lenti-TH-AADC-CH1 encoding TH, AADC and CH1 (blue lines, n=6 until w8 then n=3 until M9) had a significant improvement in rearing activity compared with the MPTP animals that received Lenti-lacZ (red lines, n=6 until w8 then n=3 until M9) or no viral injection (grey lines, n=6 until w8 then n=3 until M9). Behavioural benefit was sustained up to 9 months post Lenti-TH-AADC-CH1 injection compared to MPTP and MPTP-Lenti-lacZ control animals. One MPTP-Lenti-TH-AADC-CH1 animal was followed 30 months after lentiviral injection, and showed stable rearing correction.
   w, week after gene transfer
   M, Month after gene transfer
   **p< 0.01 relative to Normal pre-MPTP lesion state, *p< 0.05 relative to MPTP-Lenti-lacZ and to MPTP-long term animals. All data are expressed as mean ± s.e.m.
**Figure 8****: Neurodegeneration in *substantia nigra pars compacta* (SNpc) following systemic administration of neurotoxin MPTP**
   Compared to normal macaques, MPTP macaques had profound cellular loss in their SNpc (cresyl violet), indicating massive loss of dopaminergic TH-ir neurons (TH-ir), and resulting in metabolic hypoactivity as assessed by [¹⁴C]-2-deoxyglucose ([¹⁴C]-2DG) functional imaging.
**Figure 9****: dopamine transporter (DAT) immunoreactivity**
   Photomicrographs of dopamine transporter (DAT) immunoreactivity showing dramatic and equivalent dorsolateral striatal denervation in MPTP-Lenti-TH-AADC-CH1 and MPTP-Lenti-lacZ animals, as compared to. Cd: Caudate nucleus; Put: Putamen.
**Figure 10****: Neurotropism of EIAV lentiviral vector**
   Confocal microscopic images through putamen stained for (a) NeuN, (b) β-Gal and (c) the composite image. Note the yellow appearing cells in C, denoting those cells that coexpress β-Gal and NeuN, indicating that the lentivirus has transduced these neurons
**Figure 11****: *postmortem* whole tissue dopamine [DA]**_{wt} Diagrammatic representation of dopamine concentrations measured in punches taken from putamen-associated brain regions. Samples were taken from Lenti-lacZ injected (gray bars) and Lenti-TH-AADC-CH1 injected (blue bars) MPTP macaques post-mortem. [* represents a significant increase in dopamine levels compared with controls (p<0.05, n=3)].
**Figure 12****: *in vivo* localization of microdialysis probes using T2* MRI**
**Figure 13****: Stereological count of SNpc neurons after MPTP intoxication**
   Diagrammatic representation of stereological count of SNpc neurons showed no statistical difference between Lenti-TH-AADC-CH1 group (blue bar) and Lenti-lacZ group (gray bar) (n=3; KW p<0.001; *Post-hoc* MW p<0.001). [SNpc: Substantia Nigra pars compacta; VTA, ventral tegmental area, Rn: red nucleus; ns: non statistically significant].

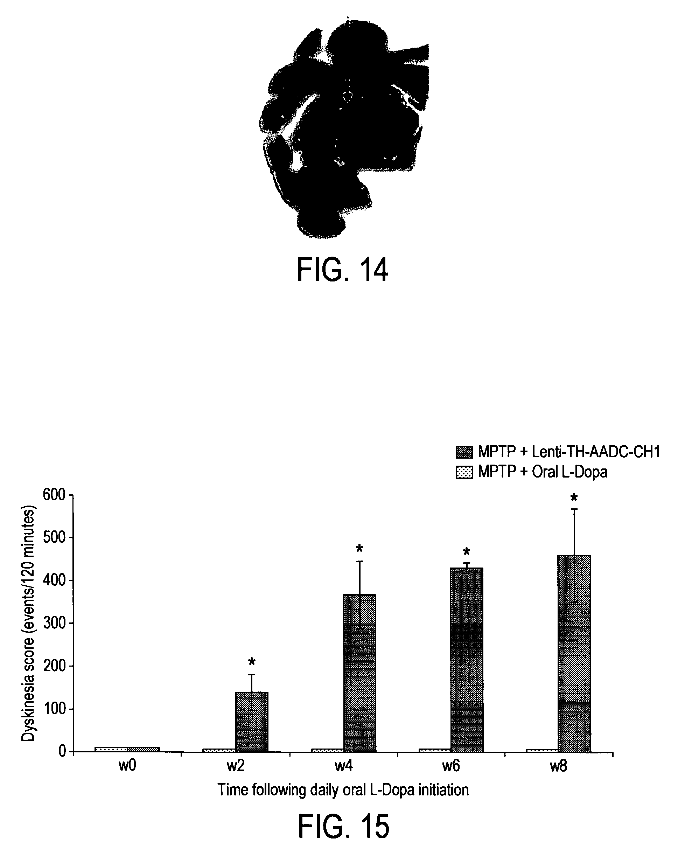

**Figure 14****: Needle tracks following MRI guided striatal injections**
   **a** *in vivo* T2* MRI study
   **b** Post-mortem analysis of needle tracts within the postcommissural, dorsal, 'motor' striatum, using Nissl histological analysis (arrow)
**Figure 15****: Gene transfer safety : Inflammation markers following gene transfer**

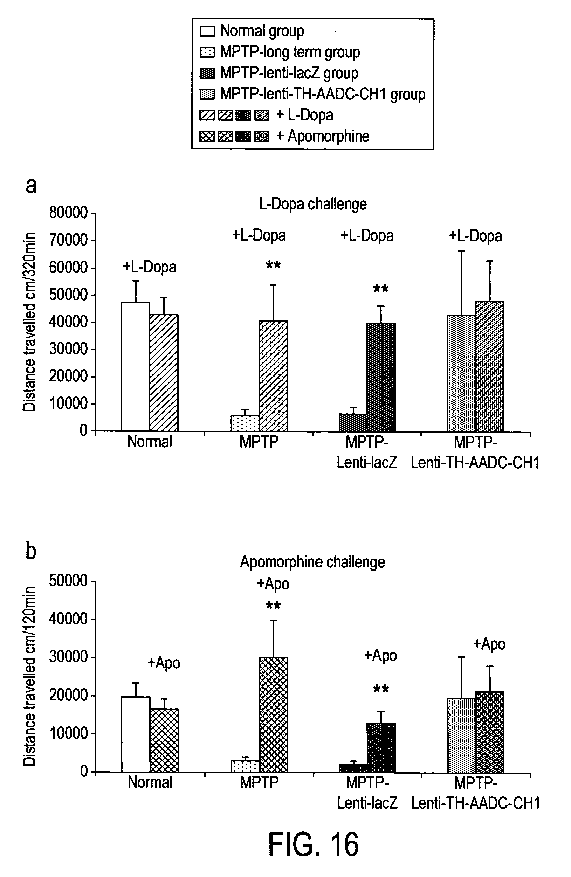

**Figure 16****: Gene transfer safety : *in vivo* MRI study**

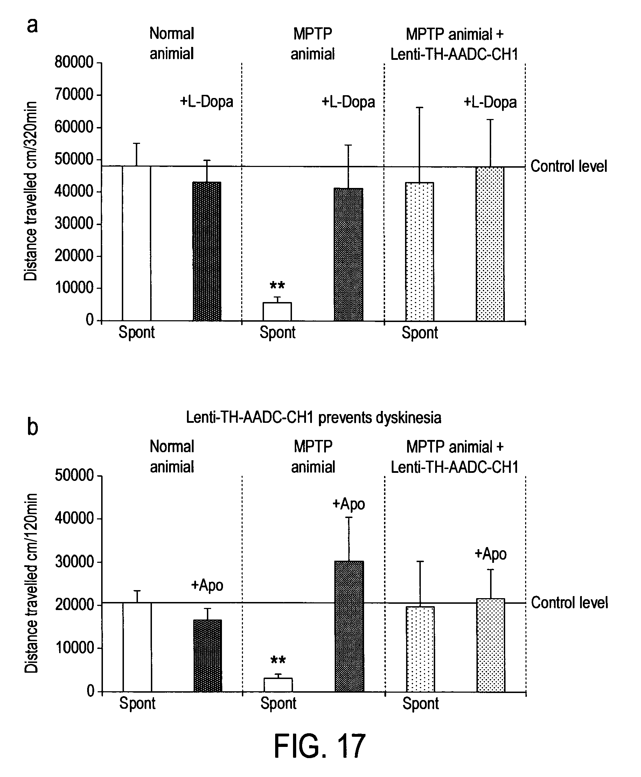

**Figure 17****: Travelled distance following pharmacological challenge**
   Using pharmacological manipulation of the dopaminergic system, the interaction between endogenous levels of dopamine and exogenous dopaminergic agents were studied (L-Dopa or Apomorphine).
   a Both systemic administration of L-Dopa and injection of Lenti-TH-AADC-CH1 (without adding L-Dopa) significantly improved motor activity in drug-naive MPTP primates, to the level of normal primate activity. Adding oral L-dopa to Lenti-TH-AADC-CH1 injected animals did not alter significantly their motor behaviour, in a similar fashion to normal unlesioned animals.
   a Acute systemic administration of a pro-dyskinetic short-acting D1/D2 dopaminergic agonist (apomorphine), induced hyperkinetic behaviour with numerous dyskinetic movements such as chorea and dystonia, in drug naive MPTP animals. By contrast, normal unlesioned animals, as MPTP Lenti-TH-AADC-CH1 animals, did not show any evidence of dyskinetic movements.
   Spont, spontaneous motor activity as measured without any drug administration. Apo, apomorphine administration. Arrows indicate excessive motor activity in MPTP animals challenged with apomorphine, which correlated to dyskinesia induction in these animals. ** p<0.01 relative to motor activity in MPTP animals after apomorphine administration; * p<0.05 relative to spontaneous motor activity in MPTP animals; N.S., not statistically significant
**Figure 18****: Reversal of L-Dopa induced dyskinesia in MPTP primates treated with Lenti-TH-AADC-CH1**

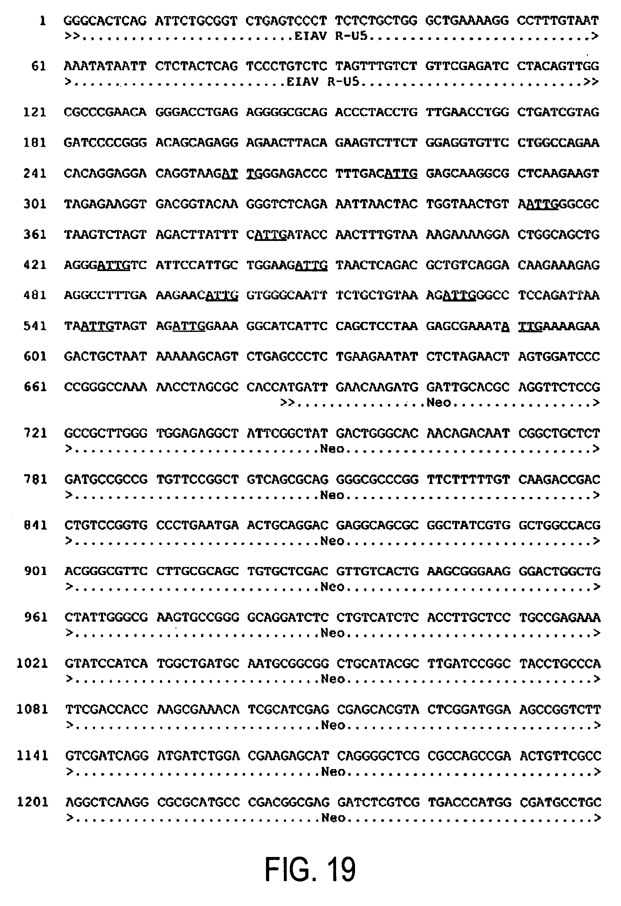

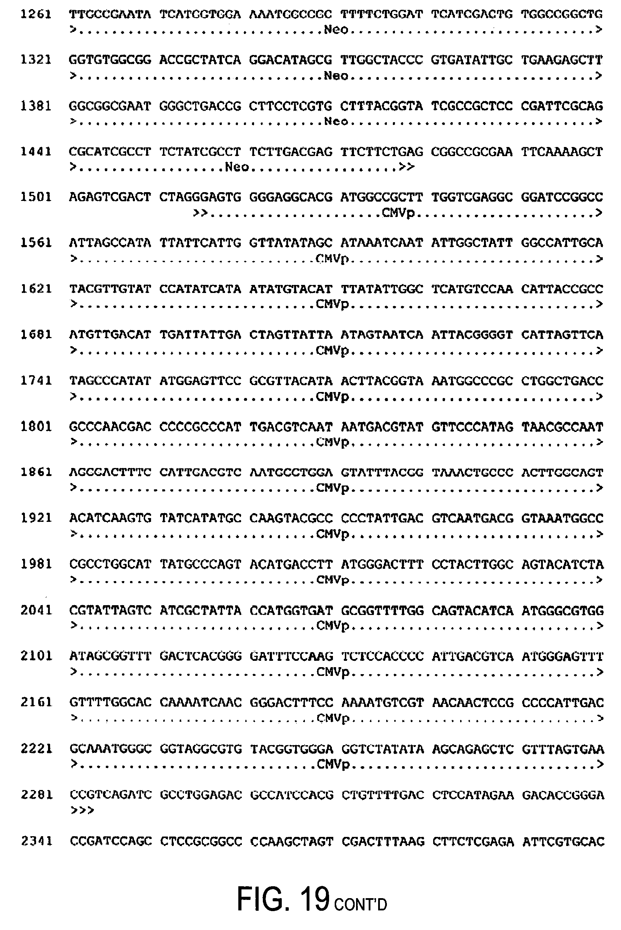

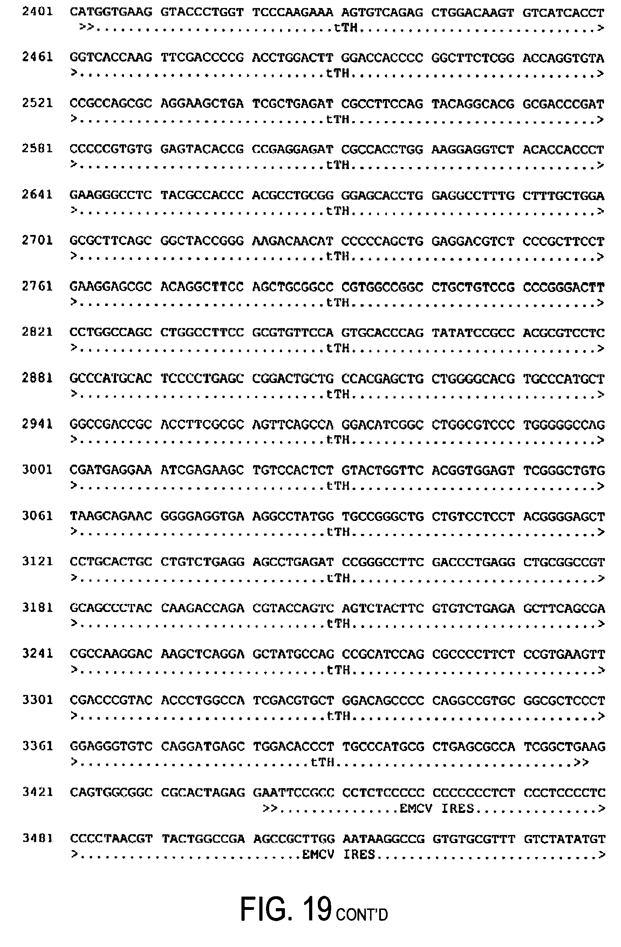

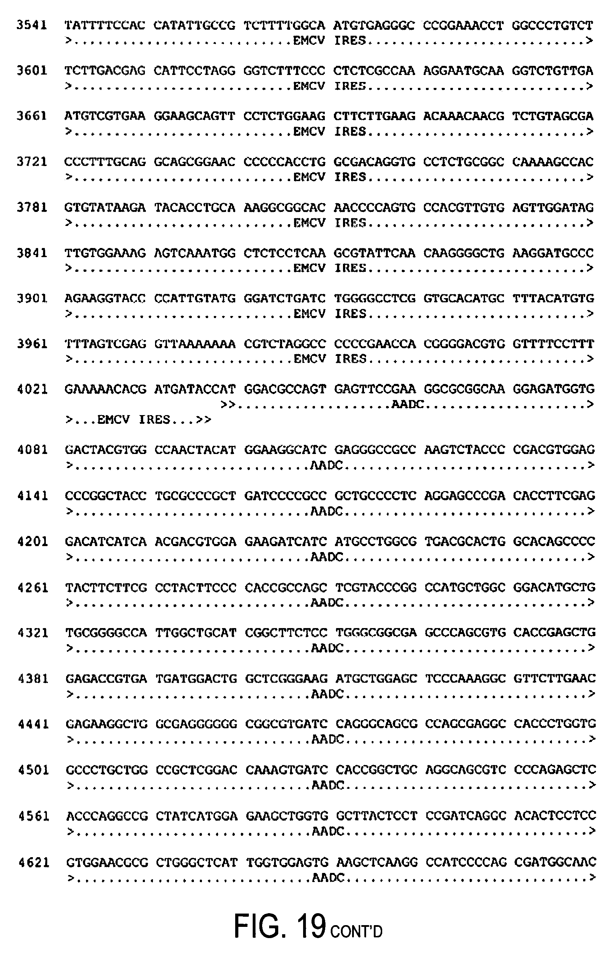

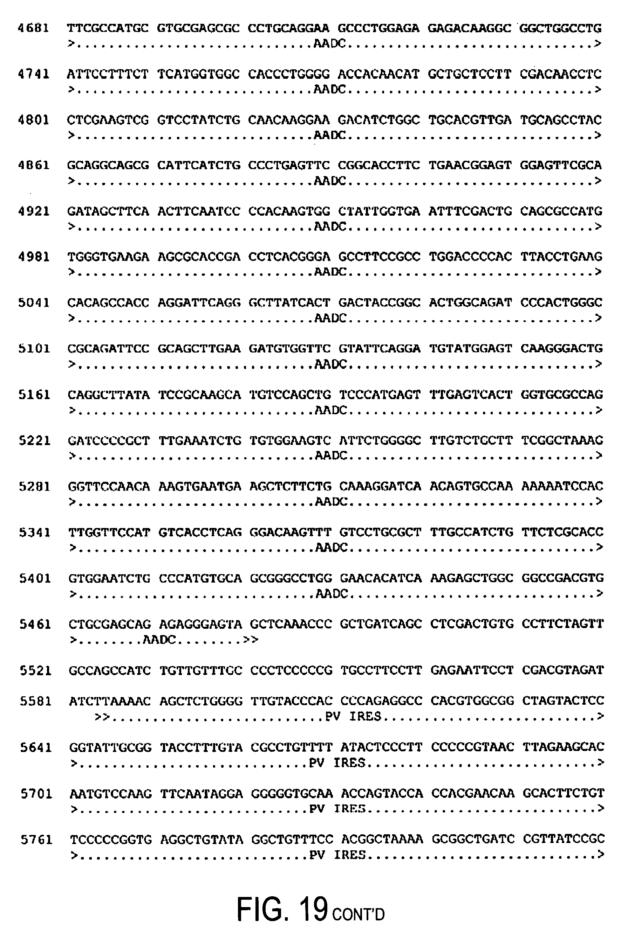

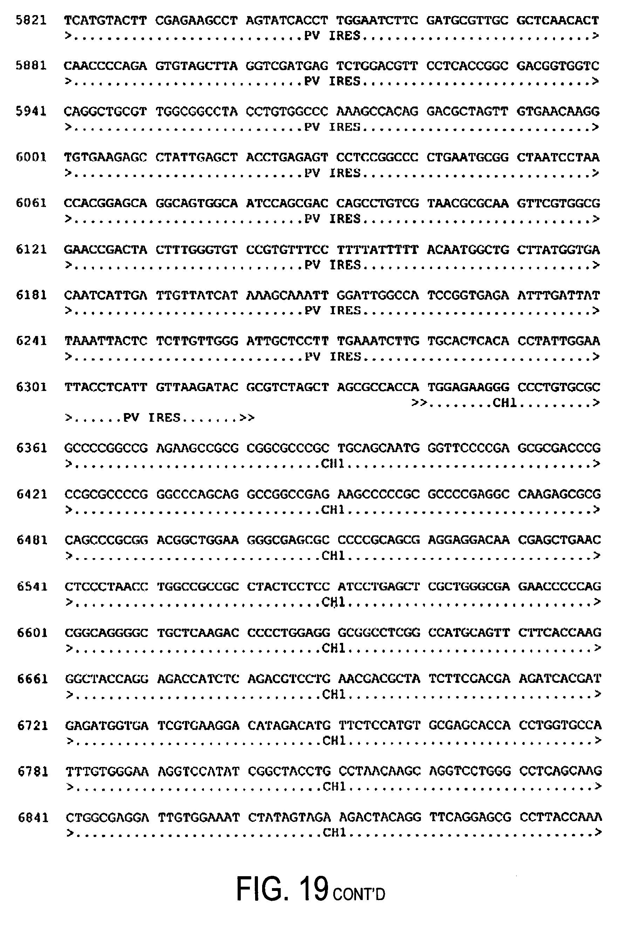

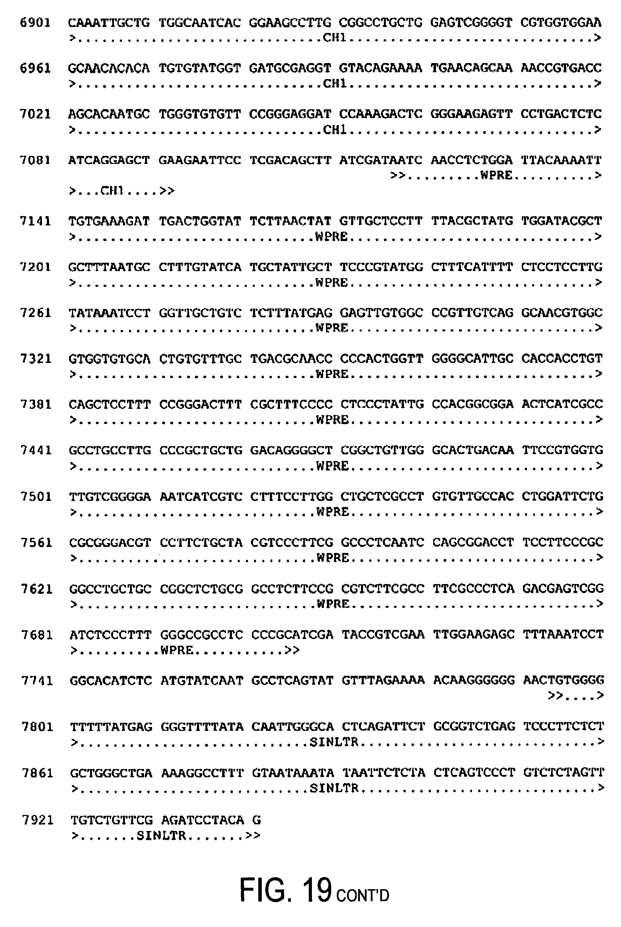

**Figure 19****: Partial sequence of Lenti-TH-AADC-CH1 (pONY8.9.4TY)**
   The nucleotide sequence of pONY8.9.4TY from the start of the EIAV R region to the end of the SIN LTR. The sequences underlined indicate the modifications in gag. These have been changed from ATG to ATTG. Neo denotes the Neomycin phosphotransferase ORG; CMVp denotes the human cytomegalovirus immediate-early enhancer/promoter; tTH denotes the truncated codon optimised tyrosine hydroxylase ORF; AADC denotes the codon optimised aromatic L-amino acid decarboxylase ORF; CH1 denotes the codon optimised GTP cyclohydrolase 1 ORF; WPRE denotes the woodchuck hepatitis virus post-transcriptional regulatory element; SINLTR denotes the self-inactivating EIAV LTR.

### DETAILED DESCRIPTION

### PARKINSON'S DISEASE

Parkinson's disease (PD) is a neurodegenerative disorder characterized by the loss of the nigrostriatal pathway. Although the cause of Parkinson's disease is not known, it is associated with the progressive death of dopaminergic (tyrosine hydroxylase (TH) positive) mesencephalic neurons, inducing motor impairment. The characteristic symptoms of Parkinson's disease appear when up to 70% of TH-positive nigrostriatal neurons have degenerated.

There is currently no satisfactory cure for Parkinson's disease. Symptomatic treatment of the disease-associated motor impairments involves oral administration of dihydroxyphenylalanine (L-DOPA). L-DOPA is transported across the blood-brain barrier and converted to dopamine, partly by residual dopaminergic neurons, leading to a substantial improvement of motor function. However, after a few years, the degeneration of dopaminergic neurons progresses, the effects of L-DOPA are reduced and side-effects appear.

An alternative strategy for therapy is neural grafting, which is based on the idea that dopamine supplied from cells implanted into the striatum can substitute for lost nigrostriatal cells. Clinical trials have shown that mesencephalic TH positive neurons obtained from human embryo cadavers (aborted foetuses) can survive and function in the brains of patients with Parkinson's disease. However, functional recovery has only been partial, and the efficacy and reproducibility of the procedure is limited (Lindvall O. & Bjorklund A. (2004) NeuroRx 1: 382-393; Kordower J.H. et al (2008) Nature Med 14: 504-506; Li J-Y et al (2008) Nature Med 14: 501-503; Braak H. & Del Tredici K. (2008) Nature Med 14: 483-485).

A further alternative strategy for therapy is gene therapy. It has been suggested that gene therapy could be used in Parkinson's disease in two ways: to replace dopamine in the affected striatum by introducing the enzymes responsible for L-DOPA or dopamine synthesis (for example, tyrosine hydroxylase); and to introduce potential neuroprotective molecules that may either prevent the TH-positive neurons from dying or stimulate regeneration and functional recovery in the damaged nigrostriatal system (Dunnet S.B. and Björklund A. (1999) Nature 399 A32-A39).

### GENE THERAPY

Gene therapy is the prevention and/or treatment of disease by introducing, replacing, altering, or supplementing a prophylactic or therapeutic gene in a subject.

Gene therapy is a powerful means to deliver proteins continuously to the central nervous system in a site-specific manner.

The present disclosure relates to dopamine gene therapy, in which one or more the genes responsible or related to dopamine synthesis is introduced into the subject.

In vivo, dopamine is synthesised from tyrosine by two enzymes, tyrosine hydroxylase (TH) and aromatic amino acid DOPA-decarboxylase (AADC). In the dopamine gene therapy method of the present invention, the vector system is preferably capable of delivering a nucleic acid sequence(s) encoding TH and AADC. The sequences of both genes are available: Accession Nos. X05290 and M76180 respecively.

The vector system used in the invention may comprise a truncated form of the TH gene, lacking the regulatory domain. The truncated TH avoids end-product feedback inhibition by dopamine (Wu J. et al (1992) 267: 25754-25758).

Functional activity of tyrosine hydroxylase depends on the availability of its cofactor tetrahydrobiopterin (BH₄). The level of cofactor may be low in the denervated striatum, and so it may be preferable if the vector system is also capable of delivering GTP cyclohydrolase I (CH1), the enzyme that catalyses the rate limiting step on the pathway of BH₄-synthesis, to ensure that sufficient levels of L-DOPA are produced *in vivo.* The sequence of the CH1 gene is also available: Accession No. U19523.

The vector system may also be capable of delivering a nucleic acid sequence encoding Vesicular Monoamine Transporter 2 (VMAT2 - Accession number L23205.1).

Dopamine replacement gene therapy may therefore involve the use of a vector system to deliver genes encoding one or more of the following genes to a subject: TH, AADC, CH1 and/or VMAT2 to the subject. The vector system may, for example deliver genes encoding TH, AADC, and CH1 to the subject. Such a vector system is described in WO 02/29065.

The vector may alternatively or also comprise a gene encoding a growth factor capable of blocking or inhibiting degeneration in the nigrostriatal system. An example of such a growth factor is a neurotrophic factor. For example the gene may encode glial cell-line derived neurotrophic factor (GDNF), brain-derived neurotrophic factor (BDNF), nerve growth factor (NGF), persephin growth factor, artemin growth factor, or neurturin growth factor, cilliary neurotrophic factor (CNTF), neurotrophic-3 (NT-3), neurotrophin-4 (NT-4) and/or pantropic neurotrophin.

The vector may alternatively or also comprise a gene encoding a neuroprotective factor. In particular, the NOI(s) may encode molecules which prevent TH-positive neurons from dying or which stimulate regeneration and functional recovery in the damaged nigrostriatal system.

### VECTOR SYSTEM

In the dopamine replacement gene therapy method of the present disclosure, the genes involved in dopamine synthesis are delivered to the subject by a vector system, such as a viral vector system.

In the context of the present invention, the terms "vector system", "vector" and "vector particle" are used synonymously to mean an entity capable of transducing a target cell with one or more nucleotides of interest (NOIs).

The concept of using viral vectors for gene therapy is well known (Verma and Somia (1997) Nature 389:239-242).

Vector systems may be based on a retrovirus, such as: murine leukemia virus (MLV), human immunodeficiency virus (HIV), equine infectious anaemia virus (EIAV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV) and all other retroviridiae including lentiviruses.

The vector system used in the invention is based on a lentivirus. Lentiviruses have the advantage that they can infect both dividing and non-dividing cells.

Examples of primate lentiviruses include the human immunodeficiency virus (HIV), the causative agent of human acquired immunodeficiency syndrome (AIDS), and the simian immunodeficiency virus (SIV). The non-primate lentiviral group includes the phototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

A retroviral vector particle may be made by a producer cell, for example, one in which the necessary genes have been introduced by a "tripe transfection" method. In this approach, the three different DNA sequences that are required to produce a retroviral vector particle i.e. the *env* coding sequences, the *gag-pol* coding sequence and the defective retroviral genome containing one or more NOIs (for example, capable of encoding one or more enzymes involved in dopamine synthesis) are introduced into the cell at the same time by transient transfection. WO 94/29438 describes the production of producer cells *in vitro* using this multiple DNA transient transfection method.

The gag-pol sequence may be codon optimised for use in the producer cell (see below).

The env protein encoded by the nucleotide sequence transfected into the producer cell may be a homologous retroviral or lentiviral env protein. Alternatively, it may be a heterologous env, or an env from a non-retro or lentivirus (see below under "pseudotyping").

The vector system used in the present invention may be a self-inactivating (SIN) vector system.

By way of example, self-inactivating retroviral vector systems have been constructed by deleting the transcriptional enhancers or the enhancers and promoter in the U3 region of the 3' LTR. After a round of vector reverse transcription and integration, these changes are copied into both the 5' and the 3' LTRs producing a transcriptionally inactive provirus. However, any promoter(s) internal to the LTRs. in such vectors will still be transcriptionally active. This strategy has been employed to eliminate effects of the enhancers and promoters in the viral LTRs on transcription from internally placed genes. Such effects include increased transcription or suppression of transcription. This strategy can also be used to eliminate downstream transcription from the 3' LTR into genomic DNA. This is of particular concern in human gene therapy where it may be important to prevent the adventitious activation of an endogenous oncogene.

A recombinase assisted mechanism may be used which facilitates the production of high titre regulated vectors from producer cells.

As used herein, the term "recombinase assisted system" includes but is not limited to a system using the Cre recombinase / loxP recognition sites of bacteriophage PI or the site-specific FLP recombinase of *S*. *cerevisiae* which catalyses recombination events between 34 bp FLP recognition targets (FRTs).

The site-specific FLP recombinase of *S. cerevisiae* which catalyses recombination events between 34 bp FLP recognition targets (FRTs) has been configured into DNA constructs in order to generate high level producer cell lines using recombinase-assisted recombination events. A similar system has been developed using the Cre recombinase / loxP recognition sites of bacteriophage P1. This was configured into a lentiviral genome such that high titre lentiviral producer cell lines were generated.

By using producer/packaging cell lines, it is possible to propagate and isolate quantities of retroviral vector particles (e.g. to prepare suitable titres of the retroviral vector particles) for subsequent transduction of, for example, a site of interest (such as adult brain tissue). Producer cell lines are usually better for large scale production or vector particles.

It is desirable to use high-titre virus preparations for transduction in tissues such as the brain. Techniques for increasing viral titre include using a *psi* plus packaging signal as discussed above and concentration of viral stocks.

A high-titre viral preparation for a producer/packaging cell is usually of the order of 10⁵ to 10⁷ retrovirus particles per ml. For transduction of the brain it is necessary to use very small volumes, so the viral preparation is concentrated by ultracentrifugation. Other methods of concentration such as ultrafiltration or binding to and elution from a matrix may be used.

The presence of a sequence termed the central polypurine tract (cPPT) may improve the efficiency of gene delivery to non-dividing cells. This *cis*-acting element is located, for example, in the EIAV polymerase coding region element. The genome of the vector system used in the present invention may comprises a cPPT sequence.

In addition, or in the alternative, the viral genome may comprise a translational enhancer.

The NOIs may be operatively linked to one or more promoter/enhancer elements. Transcription of one or more NOIs may be under the control of viral LTRs or alternatively promoter-enhancer elements. Preferably the promoter is a strong viral promoter such as CMV, or is a cellular constitutive promoter such as PGK, beta-actin or EF1alpha. The promoter may be regulated or tissue-specific. Such promoters may be selected from genes such as neurofilaments, nestin, parkin, dopamine receptors, tyrosine hydroxylase. Such promoters may also contain neurorestrictive suppressor sequences such as that found in the mu-opoid receptor gene. In a preferred embodiment, the promoter may be glial-specific or neuron-specific. The control of expression can also be achieved by using such systems as the tetracycline system that switches gene expression on or off in response to outside agents (in this case tetracycline or its analogues).

The vector system for use in the present invention may be pseudotyped with a heterologous env protein, for example with at least part of the rabies G protein or the VSV-G protein. Other envelopes which can be used to pseudotype retroviral vectrors include the Ross River virus envelope, the baculovirus GP64 protein, and the envelopes from Mokola, Ebola, 4070A and lymphocytic choriomeningitis virus (LCMV).

It has been demonstrated that a lentivirus minimal system can be constructed from HIV, SIV, FIV, and EIAV viruses. Such a system requires none of the additional genes *vif, vpr, vpx, vpu, tat, rev* and *net* for either vector production or for transduction of dividing and non-dividing cells. It has also been demonstrated that an EIAV minimal vector system can be constructed which does not require *S2* for either vector production or for transduction of dividing and non-dividing cells. The deletion of additional genes is highly advantageous. Firstly, it permits vectors to be produced without the genes associated with disease pathology in lentiviral (e.g. HIV) infections, such as *tat.* Secondly, the deletion of additional genes permits the vector to package more heterologous DNA. Thirdly, genes whose function is unknown, such as *S2*, may be omitted, thus reducing the risk of causing undesired effects. Examples of minimal lentiviral vectors are disclosed in WO-A-99/32646 and in WO-A-98/17815,

The delivery system used in the invention may therefore be devoid of at least *tat* and *S2* (if it is an EIAV vector system), and possibly also *vif, vpr, vpx, wpu* and *nef.*

The systems for use in the present invention may also be devoid of *rev.* Rev was previously thought to be essential in some retroviral genomes for efficient virus production. For example, in the case of EIAV, it was thought that *rev* and RRE sequence should be included. However, it has been found that the requirement for *rev* and RRE can be reduced or eliminated by codon optimisation or by replacement with other functional equivalent systems such as the MPMV system. As expression of the codon optimised *gag-pol* is REV independent, RRE can be removed from the *gag-pol* expression cassette, thus removing any potential for recombination with any RRE contained on the vector genome.

The viral genome of the vector system used in the present invention may therefore lack the Rev response element (RRE).

In a preferred embodiment, the system used in the present invention is based on a so-called "minimal" system in which some or all of the additional genes have be removed.

A vector system used in the present invention, capable of delivering genes which encode TH, AADC and CH1, may have one or more of the following features:
(i) at least one of the nucleic acid sequences lack an N-terminal tag;
(ii) at least one of the nucleic acid sequences is codon optimised;
(iii) where the vector system comprises a tricistronic cassette, the order of the genes in the tricistronic cassette is TH-AADC-CH1
(iv) at least one of the ATG potential start codons in gag is changed to ATG;
(v) a Neo expression cassette is inserted downstream of gag; and
(vi) where the vector system comprises a tricistronic cassette, a WPRE is inserted at the 3' end of the Tricistronic cassette to enhance expression.

These features are explained in more detail below.

### N-TERMINAL TAGS

Tags, such a polyhistidine tags or a FLAG™ tag are commonly used at the N-terminus of proteins to aid protein purification or detection of the protein using tag-specific antibodies. Tags may be added by inserting the protein-coding DNA into a vector which comprises a sequence encoding the tag, so that it is automatically included within the coding sequence. Alternatively, PCR may be performed with primers which have the tag-encoding sequence adjacent to the start codon.

For dopamine-replacement gene therapy, there is no need for the encoded dopamine synthesis enzymes to have N-terminal tags. The presence of N-terminal tags therefore unnecessarily increases the length of the genome.

### CODON OPTIMISATION

Codon optimisation has previously been described in WO99/41397. Different cells differ it their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available.

The genes delivered by the gene therapy system as well as components of the vector system may be codon optimised.

Many viruses, including HIV and other lentiviruses, use a large number of rare codons and by changing these to correspond to commonly used mammalian codons, increased expression of the packaging components in mammalian producer cells can be achieved. Codon usage tables are known in the art for mammalian cells, as well as for a variety of other organisms.

### Codon optimisation of gag pol

Codon optimisation has a number of other advantages. By virtue of alterations in their sequences, the nucleotide sequences encoding the packaging components of the viral particles required for assembly of viral particles in the producer cells/packaging cells have RNA instability sequences (INS) eliminated from them. At the same time, the amino acid sequence coding sequence for the packaging components is retained so that the viral components encoded by the sequences remain the same, or at least sufficiently similar that the function of the packaging components is not compromised. Codon optimisation also overcomes the Rev/RRE requirement for export, rendering optimised sequences Rev independent. Codon optimisation also reduces homologous recombination between different constructs within the vector system (for example between the regions of overlap in the gag-pol and env open reading frames). The overall effect of codon optimisation is therefore a notable increase in viral titre and improved safety.

In one embodiment only codons relating to INS are codon optimised. Alternatively sequences may be codon optimised in their entirety, with the exception of the sequence encompassing the frameshift site.

The *gag-pol* gene comprises two overlapping reading frames encoding gag and pol proteins respectively. The expression of both proteins depends on a frameshift during translation. This frameshift occurs as a result of ribosome "slippage" during translation. This slippage is thought to be caused at least in part by ribosome-stalling RNA secondary structures. Such secondary structures exist downstream of the frameshift site in the *gag-pol* gene. For HIV, the region of overlap extends from nucleotide 1222 downstream of the beginning of *gag* (wherein nucleotide 1 is the A of the *gag* ATG) to the end of *gag* (nt 1503). Consequently, a 281 bp fragment spanning the frameshift site and the overlapping region of the two reading frames is preferably not codon optimised. Retaining this fragment will enable more efficient expression of the gag-pol proteins.

For EIAV the beginning of the overlap has been taken to be nt 1262 (where nucleotide 1 is the A of the *gag* ATG). The end of the overlap is at 1461 bp. In order to ensure that the frameshift site and the *gag-pol* overlap are preserved, the wild type sequence has been retained from nt 1156 to 1465.

Derivations from optimal codon usage may be made, for example, in order to accommodate convenient restriction sites, and conservative amino acid changes may be introduced into the gag-pol proteins.

Due to the degenerate nature of the Genetic Code, it will be appreciated that numerous *gag-pol* sequences can be achieved by a skilled worker. Also there are many retroviral variants described which can be used as a starting point for generating a codon optimised *gag-pol* sequence. Lentiviral genomes can be quite variable. For example there are many quasi-species of HIV-1 which are still functional. This is also the case for EIAV. These variants may be used to enhance particular parts of the transduction process. Examples of HIV-1 variants may be found at http://hiv-web.lanl.gov. Details of EIAV clones may be found at the NCBI database: http://www.ncbi.nlm.nih.gov.

The strategy for codon optimised *gag-pol* sequences can be used in relation to any retrovirus. This would apply to all lentiviruses, including EIAV, FIV, BIV, CAEV, VMR, SIV, HIV-1 and HIV-2. In addition this method could be used to increase expression of genes from HTLV-1, HTLV-2, HFV, HSRV and human endogenous retroviruses (HERV), MLV and other retroviruses.

Codon optimisation can render *gag-pol* expression Rev independent. In order to enable the use of anti-*rev* or RRE factors in the retroviral vector, however, it would be necessary to render the viral vector generation system totally Rev/RRE independent Thus, the genome also needs to be modified. This is achieved by optimising vector genome components. Advantageously, these modifications also lead to the production of a safer system absent of all additional proteins both in the producer and in the transduced cell.

As described above, the packaging components for a retroviral vector include expression products of *gag, pol* and *env* genes. In addition, efficient packaging depends on a short sequence of 4 stem loops followed by a partial sequence from *gag* and *env* (the "packaging signal"). Thus, inclusion of a deleted *gag* sequence in the retroviral vector genome (in addition to the full *gag* sequence on the packaging construct) will optimise vector titre. To date efficient packaging has been reported to require from 255 to 360 nucleotides of *gag* in vectors that still retain *env* sequences, or about 40 nucleotides of *gag* in a particular combination of splice donor mutation, *gag* and *env* deletions. It has surprisingly been found that a deletion of all but the N-termnial 360 or so nucleotides in *gag* leads to an increase in vector titre. Thus, preferably, the retroviral vector genome includes a *gag* sequence which comprises one or more deletions, more preferably the gag sequence comprises about 360 nucleotides derivable from the N-terminus.

### MODIFICATION OF POTENTIAL START CODONS IN GAG

To ensure that translation begins from the correct start codon (ATG), upstream start codons in gag may be manipulated. Conveniently, upstream start codons are mutated by substitution e. g. ATG to ACG or insertion ATG to ATTG by techniques known in the art.

This ensures that the first available ORF of the mature mRNA in the target cells will be for the therapeutic gene.
In the context of the present invention, at least one potential start codon, preferably all the potential start codons in gag are mutated.

### INSERTION OF A NEO EXPRESSION CASSETTE

Insertion of an open reading frame, or part thereof, downstream of a viral LTR and downstream of an internal promoter has been shown to enhance viral titre in the absence of rev (as described in WO 03/064665). Thus in a preferred embodiment, a Neo-expression cassette is inserted downstream of gag.

### WPRE

The viral genome may comprise a post-translational regulatory element. For example, the genome may comprise an element such as the woodchuck hepatitis virus posttranscriptional regulatory element (WPRE), such as that described in US 2005/0002907.

A vector system may comprise a plurality of vectors, each capable of delivering a gene encoding an enzyme involved in dopamine synthesis.

An alternative strategy is to deliver all three genes to target cells using a single vector.

### LENTIVIRAL VECTOR SYSTEMS

WO 02/29065 describes a Tricistronic lentiviral vector capable of delivering genes encoding tyrosine hydroxylase (TH), aromatic L-amino acid decarboxylase (AADC), and GTP cyclohydrolase 1 (CH1) to a host cell. It is shown that expression of there enzymes causes production of dopamine, L-Dopa and DOPAC in cells in culture and is therapeutically effective against a rodent model of parkinson's disease.

### ADENO-ASSOCIATED VECTORS

Adeno-associated virus vectors (AAVs) have also been used to deliver to the brain, genes associated with dopamine synthesis. The use of separate AAV vectors to transfer two or three critical genes has demonstrated some behavioural benefit in rat and non-human primate (NHP) models of PD. (Kirik et al (2002) PNAS 99:4708-4713; Muramatsu et al (2002) Human Gene Therapy 13:345-354).

It was previously thought that delivery of three genes (for example genes encoding AADC, TH and GCH-1) to striatal cells using a single AAV vector would not be achievable due to the packaging restraints of these vectors (Kirik et al (2002) as above; Muruamatsu et al (2002) as above; Shen et al (2000) Human Gene Therapy 11:1509-1519; Sun et al (2004) Human Gene Therapy 15:1177-1196; Carlsson et al (2005) Brain 128:559-569).

However, recent reports have demonstrated that certain AAV vectors can efficiently incorporate large payloads (up to 8.9kb). The most efficient of these vectors was found to have an AAV5 capsid and an AAV2 ITR (Allocca M. et al J.Clin Invest. (2008) 118: 1955-1964).

### INTERNAL RIBOSOME ENTRY SITE (IRES)

The viral genome of the vector system used in the invention comprises three or more NOIs. In order for the NOIs to be expressed, there may be three or more transcription units within the vector genome, one for each NOI. Retroviral vectors achieve the highest titres and most potent gene expression properties if they are kept genetically simple, and so it is preferable to use an internal ribosome entry site (IRES) to initiate translation of the second (and subsequent) coding sequence(s) in a poly-cistronic message.

Insertion of IRES elements into retroviral vectors is compatible with the retroviral replication cycle and allows expression of multiple coding regions from a single promoter. IRES elements were first found in the non-translated 5' ends of picornaviruses where they promote cap-independent translation of viral proteins. When located between open reading frames in an RNA, IRES elements allow efficient translation of the downstream open reading frame by promoting entry of the ribosome at the IRES element followed by downstream initiation of translation.

A number of different IRES sequences are known including those from encephalomyocarditis virus (EMCV); BiP protein; the *Antennapedia* gene of Drosophila (exons d and e) as well as those in polio virus (PV).

According to WO-A-97/14809, IRES sequences are typically found in the 5' noncoding region of genes. In addition to those in the literature they can be found empirically by looking for genetic sequences that affect expression and then determining whether that sequence affects the DNA (i.e. acts as a promoter or enhancer) or only the RNA (acts as an IRES sequence).

The term "IRES" includes any sequence or combination of sequences which work as or improve the function of an IRES.

The IRES(s) may be of viral origin (such as EMCV IRES, PV IRES, or FMDV 2Alike sequences) or cellular origin (such as FGF2 IRES, NRF IRES, Notch 2 IRES or EIF4 IRES).

In order for the IRES to be capable of initiating translation of each NOI, it should be located between or prior to NOIs in the vector genome. For example, for a multicistronic sequence containing n NOIs, the genome may be as follows:

[(NOI₁ -IRES₁]...NOIₙ n=1→n

For bi and tri-cistronic sequences, the order may be as follows:

NOI₁-IRES₁-NOI₂

NOI₁-IRES₁-NOI₂-IRES₂-NOI₃

Alternative configurations of IRESs and NOIs can also be utilised. For example transcripts containing the IRESs and NOIs need not be driven from the same promoter.

An example of this arrangement may be:

IRES₁-NOI₁-promoter-NOI₂-IRES₂-NOI₃.

In any construct utilising an internal cassette having more than one IRES and NOI, the IRESs may be of different origins, that is, heterologous to one another. For example, one IRES may be from EMCV and the other IRES may be from polio virus.

IRESs are also suitable for use with AAV and adenoviral vectors.

### PHARMACEUTICAL COMPOSITIONS

The vector for dopamine replacement gene therapy used in the present invention may be present in a pharmaceutical composition, wherein the composition comprises a prophylactically or therapeutically effective amount of the vector.

The methods of the disclosure may be used to treat and/or prevent Parkinson's disease in a subject.

'Treating' as used herein refers to treatment of a subject having a disease in order to ameliorate, cure or reduce the symptoms of the disease, or reduce or halt the progression of the disease. For example, the method of the disclosure may improve or restore motor function, and/or reduce dyskinesias. Patients treated with dopamine replacement gene therapy as described herein may continue to be treated with L-dopa, which may be at a reduced dose to that taken prior to dopamine replacement therapy.

The term 'preventing' is intended to refer to averting, delaying, impeding or hindering the contraction of a disease. For example, the method of the disclosure may delay or stop the progressive death of mesencephalic neurons, thus preventing motor impairment. The method of the disclosure may reduce the likelihood of dyskinesias.

The pharmaceutical composition may optionally comprise a pharmaceutically acceptable carrier, diluent or excipient.

The composition may be administered by injection. For example, the composition may be administered by injection into the caudate putamen. In the examples given herein, the composition is administered by bilateral injections into each motor putamen.

The vector system may be provided in the form of a kit together with needles and/or catheters used to administer the vectors to the brain.

The dopamine replacement gene therapy methods of the present disclosure may be use in conjunction with dopamine therapy. As shown in the Examples presented herein, gene therapy using a lentiviral vector expressing TH, AADC and CH1 shows a synergistic effect with L-Dopa treatment.

L-Dopa may be administered by any convenient means, such as orally or by intramuscular injection, and may be prior to or contemporanous with dopamine replacement gene therapy.

### DYSKINESIAS

Dyskinesia is the impairment of the power of voluntary movement, resulting in fragmentary or incomplete movements.

Dyskinesias are a common side-effect of chronic L-Dopa intake. In many cases patients cycle between ON-drug periods, which are complicated with abnormal involuntary movements (dyskinesias), and OFF-drug periods where the patients are akinetic (muscle rigidity).

It is thought that dyskinesias are at least partly caused by intermittent oral uptake of L-Dopa and consequent pulsatile stimulation of striatal dopamine receptors.

Becuase the vector system used in the invention replaces dopamine by gene therapy, continuous delivery of dopamine should be achieved which maintains or restores constant dopaminergic tone in the stratum.

Dyskinesias associated with abberations in striatal tone in the subject should therefore be avoided.

Dopaminergic tone may be achieved at physiological levels or subphysiological levels (see below).

It has surprisingly been shown that dopamine replacement gene therapy is not only able to avoid dyskinesias associated with L-Dopa treatment, but to provide therapy for patients who have already developed dyskinesias from long-term L-Dopa treatment. Thus dopamine replacement gene therapy can prevent subsequent occurrences of dyskinesia following oral L-Dopa therapy.

The present disclosure thus also provides a method for treating and/or preventing dyskinesias associated with oral L-dopa administration in a Parkinson's disease subject by administration of a vector system for dopamine replacement gene therapy to the subject.

### DOPAMINE LEVELS FOLLOWING GENE THERAPY

The gene therapy strategy used in the present invention may induce dopamine synthesis in the striatum such that levels of dopamine are achieved which are higher than those associated with the untreated PD striatum, but lower than those associated with the non-PD striatum. The vector system may cause the production of sub-physiological levels of dopamine in the striatum.

Production of sub-physiological levels of dopamine has been shown by the present inventors to be therapeutically effective in a primate model of PD. Production of sub-physiological levels is preferable to the production of super-physiological levels as "over-dosing" with dopamine can be harmful, for example because it may induce dyskinesias. Also, over-production of the dopamine-producing enzymes may be harmful for the host cell.

Production of lower levels of enzymes also necessitates administration of less of the vector system for gene therapy, reducing costs and minimising any adverse effect associated with the treatment.

### DOPAMINE TRANSPORTER

The Dopamine Transporter or Dopamine Active Transporter (DAT) is an integral membrane protein localized in the plasma membrane of synaptic terminals of dopaminergic neurons of the substantia nigra which are located in the striatum. The DAT removes recycles dopamine from the synaptic cleft, terminating the dopamine signal.

Increased levels of DAT are associated with depression and other disorders such as ADHD.

Without wishing to be bound by theory, the present inventors predict that one of the reasons for failure of stem cell/foetal tissue grafting in the substantia nigra to induce dopamine synthesis, is the presence of DAT in the grafted tissue. DAT may act to sequester extracellular dopamine released from the grafted tissue so that it cannot perform its physiological effect.

The method of the present disclosure involves increasing the efficacy of dopamine gene therapy by avoiding sequestration of dopamine by DAT.

Sequestration of dopamine by DAT may conveniently be avoided by administration of the vector system for gene therapy to a tissue which lacks significant DAT expression.

A tissue is considered "to lack significant DAT expression" if the levels of DAT are sufficiently low that they do not significantly interfere with dopamine produced by the dopamine replacement gene therapy strategy.

DAT levels significantly interfere with the dopamine replacement gene therapy strategy if is sequesters dopamine to such an extent that effects the prophylactic and/or therapeutic effect of the treatment.

DAT levels are decreased in striatum of Parkinson's patients. Sequestration of dopamine by DAT may therefore be avoided by expressing the enzymes involved in dopamine synthesis in the striatum of a Parkinson's patient.

### DAT INHIBITORS

An alternative or additional approach to administration to a low- or no-DAT tissue is to inhibit sequestration of dopamine by DAT by using one or more DAT inhibitors.

A DAT inhibitor may inhibit the expression or activity of DAT.

The gene for DAT is located on chromosome 5p15. The protein encoding region of the gene is over 64 kb long and is comprised of 15 exons.

Nurr1, a nuclear receptor that regulates many dopamine related genes, can bind the promoter region of this gene and induce expression. This promoter may also be the target of the transcription factor Sp-1.

Expression of DAT may be inhibited by various methods known in the art, such as by antisense or RNAi technology against the DAT gene or Nurr 1.

For example, the DAT inhibitor may comprise an antisense nucleic acid molecule or an inhibitory RNA specific to the DAT sequence. The inhibitor may also be a micro RNA which binds to a target sequence in the DAT gene and thereby prevent expression of DAT.

While transcription factors control which cells express DAT, functional regulation of this protein is largely accomplished by kinases. Both MAPK and PKC can modulate the rate at which the transporter moves dopamine or cause the internalization of DAT.

The function of DAT may therefore be inhibited by modulation of the expression and/or activity of one or more kinase enzymes.

A number of DAT inhibitors are known, including methylphenidate, cocaine, bupropion and Ritalin.

In the context of the present invention, the DAT inhibitor may be administered to the subject before dopamine replacement gene therapy or simultaneously.

The vector system and the DAT inhibitor (or its in vivo production system) may be provided in the form of a kit for separate, sequential, combined or simultaneous administration to a subject. The vector system for use according to the present invention could also deliver an inhibitory RNA in addition to the enzymes involved in dopamine replacement therapy.

### SIRNA/MICRO RNA

The vector system may comprise or encode a siRNA or micro-RNA or shRNA or regulated micro or shRNA (Dickins et al. (2005) Nature Genetics 37: 1289-1295, Silva et al. (2005) Nature Genetics 37:1281-1288).

Post-transcriptional gene silencing (PTGS) mediated by double-stranded RNA (dsRNA) is a conserved cellular defence mechanism for controlling the expression of foreign genes. It is thought that the random integration of elements such as transposons or viruses causes the expression of dsRNA which activates sequence-specific degradation of homologous single-stranded mRNA or viral genomic RNA. The silencing effect is known as RNA interference (RNAi) (Ralph et al. (2005) Nature Medicine 11:429-433). The mechanism of RNAi involves the processing of long dsRNAs into duplexes of about 21-25 nucleotide (nt) RNAs. These products are called small interfering or silencing RNAs (siRNAs) which are the sequence-specific mediators of mRNA degradation. In differentiated mammalian cells dsRNA >30bp has been found to activate the interferon response leading to shutdown of protein synthesis and non-specific mRNA degradation (Stark et al. (1998)). However this response can be bypassed by using 21nt siRNA duplexes (Elbashir et al. (2001), Hutvagner et al. (2001)) allowing gene function to be analysed in cultured mammalian cells.

Micro-RNAs are a very large group of small RNAs produced naturally in organisms, at least some of which regulate the expression of target genes. Founding members of the micro-RNA family are *let-7* and *lin-4.* The *let-7* gene encodes a small, highly conserved RNA species that regulates the expression of endogenous protein-coding genes during worm development. The active RNA species is transcribed initially as an ∼70nt precursor, which is post- transcriptionally processed into a mature ∼21nt form. Both *let-7* and *lin-4* are transcribed as hairpin RNA precursors which are processed to their mature forms by Dicer enzyme.

### COGNITIVE IMPAIRMENT

Oral L-Dopa treatment can be associated with cognitive impairment.

Parkinson's disease is characterised primarily by dopamine depletion in the dorsal striatum. Dopamine function in the ventral or "cognitive" striatum and the prefrontal cortex is usually unaffected. Oral administration of L-dopa stimulates all the brain dopaminergic systems, meaning that it "over-doses" the cognitive striatum and impairs associated cognitive functions.

It has surprisingly been found that, by using gene therapy to replace dopamine, both local and tonic dopamine levels are restored. In other words, dopamine replacement gene therapy elevates dopamine levels in the dorsal striatum without over-raising dopamine levels in the cognitive striatum.

The methods of the present disclosure therefore treat and/or prevent Parkinson's disease without causing cognitive impairment.

### MOTOR DYSFUNCTION

Motor dysfunction associated with Parkinson's disease is thought to arise from dysfunction of the basal ganlia, the deep brain structures which control movement.

It is thought that abnormal over-activity of output nuclei such as the internal globus pallidus (GPi) is responsible.

Surprisingly it has been found that dopamine replacement gene therapy can normalise neuronal activities in the basal ganglia output nuclei, reducing the abnormal high firing rate of PD GPi neurons and reducing the proportion of spikes per burst and the number of burst events in the neuronal firing pattern.

Dopamine replacement gene therapy also reduces neuronal hyperactivity in the subthalamic nucleus (STN). This is detectable by looking at decreases in metabolic activity of the STN.

The present disclosure provides a method for normalising neuronal electrical activity in basal ganglia and/or subthalamic nucleus in a Parkinson's disease subject by administration of a vector system for dopamine replacement gene therapy to the subject.

The term "normalising" in this context means that the increase in the mean firing rate of GPi neurons and/or neuronal hyperactivity in the STN associated with PD is reduced. The mean firing rate and/or neuronal activity may be maintained at a normal, non-PD level, reduced to a non-PD level, or reduced such that it is still elevated compared to a non-PD individual, but still less that the level which would be expected in an individual without treatment.

Looking at the pattern of neuronal firing, administration of the vector system reduces the number of spikes per burst and/or the number of burst events in the GPi.

If the patient is treated before onset of symptoms, the vector system may normalise number of spikes per burst and/or the number of burst, such that they are not raised or not raised to the same extent that they would be in the absence of treatment.

The invention will now be further described by way of Examples, which are meant to serve to assist one of ordinary skill in the art in carrying out the invention and are not intended in any way to limit the scope of the invention.

### EXAMPLES

### EXAMPLES

### Example 1 - Long term motor behavioural restoration

To investigate the potential of gene transfer of TH, AADC and CH1 to correct Parkinsonism, a long term study was performed in the MPTP primate model of PD. A tricistronic lentiviral vector was designed that encodes the genes for TH, AADC and CH1 (Lenti-TH-AADC-CH1). One to eight weeks after the cessation of MPTP intoxication, 18 MPTP treated macaques were assigned into three behaviourally equivalent groups. The first group (MPTP-Lenti-TH-AADC-CH1, n=6) received bilateral injections of Lenti-TH-AADC-CH1 into each motor putamen. The second group (MPTP-Lenti-lacZ, n=6) received a control EIAV vector encoding the LacZ reporter gene. The third group (MPTP-*long term,* n=6) did not receive any surgical intervention but were included as an additional control to evaluate the stability of the MPTP model. All animals were maintained throughout the study without treatment using L-Dopa or dopaminergic drugs.

Animals treated with Lenti-TH-AADC-CH1 demonstrated significant improvements in akinesia and posture as early as the second week post-vector injection compared with controls (Friedman P<0.001, *Post-hoc* MW *MPTP-long term,* p< 0.05, MPTP-LacZ p<0.05) (Fig. 1, Fig. 7). Clinical observations also showed a significant improvement (decrease) in the global clinical rating scale of the MPTP-Lenti-TH-AADC-CH1 group compared with controls as soon as 6 weeks post viral injection (Friedman P<0.001, *Post-hoc* MW MPTP-*long term* p< 0.05, MPTP-LacZ p<0.05, Fig. 1a). Animals treated with MPTP Lenti-TH-AADC-CH1 continued to gradually recover from akinesia and posture impairment without any additional dopaminergic intake, reaching 85% of total distance moved (Fig. 1b) and 100% of rearing activity (Fig. 7) relative to Normal state, up to 9 months post-lentiviral injection. Animals were sacrificed at various time points during the study for supplementary analyses. The Lenti-TH-AADC-CH1 treated animal with the longest follow up time point was maintained on the study for 30 months post lentiviral treatment and demonstrated a sustained motor improvement throughout. The control MPTP-*long term* animals have remained severely disabled at all time points (Fig. 1). No Lenti-TH-AADC-CH1 treated animal demonstrated a reversal in behavioural improvement during the observation period.

### Example 2 - Local and continuous dopamine production in motor striatum

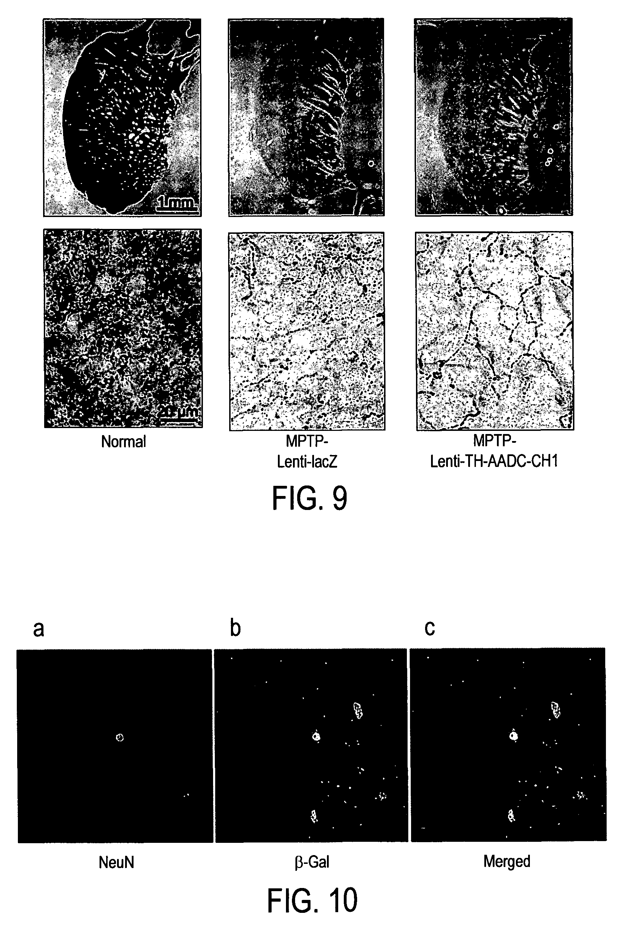

To investigate *in vivo* gene transfer and lentiviral mediated dopamine production, histological analysis of transgene expression was performed and local dopamine levels were measured in the striatum of study animals. Animals treated with Lenti-LacZ were demonstrated to have an average of 54 947 transduced cells per injected putamen, which were mostly neurons (NeuN-ir positive >90%, Fig. 10). Histological analysis also demonstrated that TH, AADC and CH1 positive neurons were evident in the vicinity of the putaminal injection site in MPTP Lenti-TH-AADC-CH1 treated animals but not in MPTP-Lenti-lacZ controls (Fig. 2).

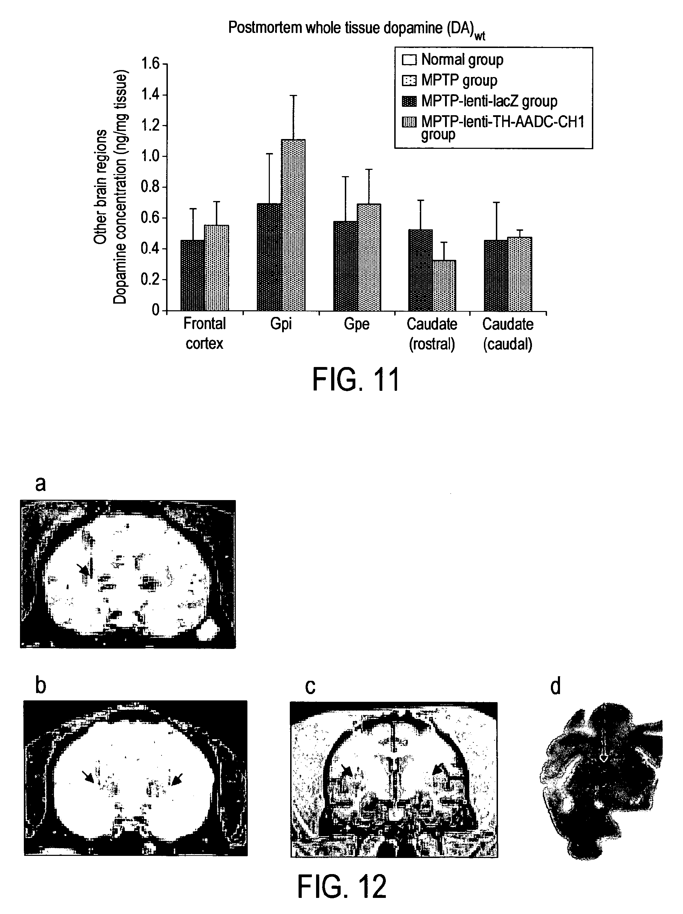

To quantitatively measure lentiviral mediated dopamine production in the putamen, two indices were applied: (1) whole tissue dopamine levels [DA]_{wt}, measured by *post-mortem* analysis of striatal punches: this index quantifies both intracellular dopamine (presynaptic nigral dopaminergic terminals) and extracellular dopamine contents; (2) extracellular dopamine levels [DA]_{ec}, measured *by in vivo* microdialysis: this index specifically quantifies the released dopamine within the extracellular milieu. To measure [DA]_{wt}, fresh brain punches were taken from each animal at the time of sacrifice. Lenti-TH-AADC-CH1 significantly increased [DA]_{wt} in dorsal putamen (Fig. 3a) compared to Lenti-LacZ. Comparison of [DA]_{wt} in putamen punches from unlesioned macaques (no MPTP treatment n=2, Fig. 3a) revealed that the level of dopamine replacement in Lenti-TH-AADC-CH1 injected putamen was 1-4% of normal levels. Although low, the degree of dopamine replacement, based on measures of [DA]_{wt}, reflects the nigral neurodegeneration that decreases presynaptic dopamine pools within the striatum (Fig. 3). The effects of Lenti-TH-AADC-CH1 were specific to the putamen region, as no unregulated release of dopamine was observed in distal brain regions, such as cortex, globus pallidus and caudate (Fig. 11). This addresses an important safety issue in terms of clinical application for this gene therapy approach.

To assess dopaminergic tone within the striatum, [DA]_{ec} levels were measured in Normal (unlesioned n=6), MPTP-*long term* (n=7), MPTP-Lenti-LacZ (n=5) and MPTP-Lenti-TH-AADC-CH1 (n=3) primates. Microdialysis probes were placed in the post-commissural putamen for each animal (see methods section, Fig. 12).

Significant [DA]_{ec} differences have been demonstrated between groups (KW p< 0.001). In the MPTP*-long term* and MPTP-Lenti-LacZ animals, baseline [DA]_{ec} was reduced to 26 % and 23 % of normal dopamine levels respectively, indicating a severe dopamine depletion in these animals (*Post-hoc* MW P<0.001, Fig. 3b). Lenti-TH-AADC-CH1 significantly increased baseline [DA]_{ec} compared to both MPTP-*long term* and MPTP-Lenti-LacZ, reaching 60 % of normal levels in the postcommissural putamen (*Post-hoc* MW P<0.05, Fig. 3b). To assess dynamic interactions between endogenous and exogenous dopamine, [DA]_{ec} was measured in each animal following intramuscular administration of L-Dopa. The result indicates that Lenti-TH-AADC-CH1 treatment appears to be synergistic with L-Dopa treatment since dopamine levels were increased from 3 918 pg/ml to 8 843 pg/ml (2.25-fold) in the Lenti-TH-AADC-CH1 animal compared with a 1697 pg/ml to 1991 pg/ml (1.17-fold) increase in the MPTP-long term animal (Fig. 3c). This could be explained by the increase AADC in the putamen mediated by Lenti-TH-AADC-CH1 gene transfer. In line with the hypothesis, levels of L-Dopa in the striatum were increased only in the MPTP-*long term* and MPTP-Lenti-LacZ group following L-Dopa injection suggesting that most of the injected L-Dopa was converted into DA in normal control and MPTP-Lenti-TH-AADC-CH1 animals (Fig. 3d).

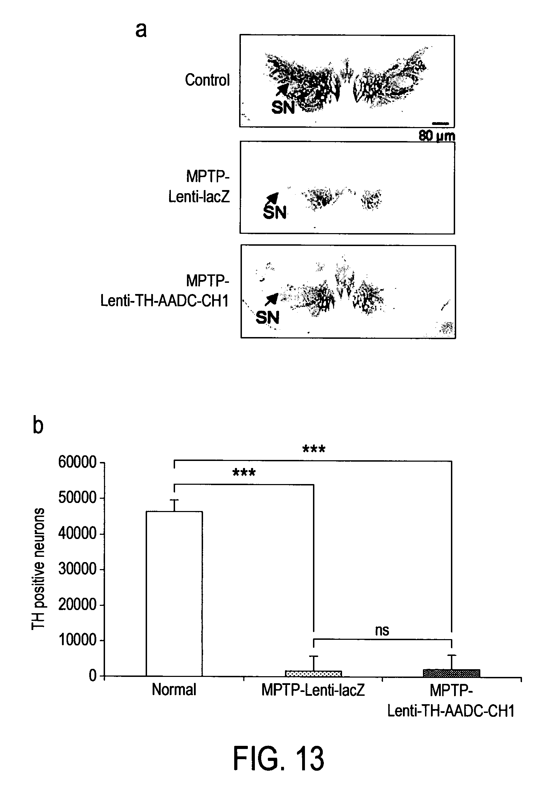

The behavioural efficacy observed in MPTP-Lenti-TH-AADC-CH1 animals was not a consequence of less efficient nigro-striatal lesioning. Stereology counts of dopaminergic neurons in the substantia nigra pars compacta (SNpc) showed a dramatic decrease in the number of TH-ir neurons of both MPTP- Lenti-TH-AADC-CH1 and MPTP-LacZ groups compared to normal animals (KW p<0.001; *Post-hoc* MW p<0.001, Fig. 13). Furthermore, no difference was observed between the number of TH-ir neurons in the two lesioned MPTP groups (*Post-hoc* MW p=0.51, Fig. S8).

### Example 3 - Restoration of basal ganglia activity

To determine the mechanism by which Lenti-TH-AADC-CH1 corrected motor dysfunction, neuronal activity was investigated within the basal ganglia system.

The current model of basal ganglia dysfunction in PD suggests that abnormal over-activity of output nuclei such as the internal globus pallidus (GPi) account for the motor symptoms observed in this disorder. To determine if Lenti-TH-AAADC-CH1 could normalize neuronal electrical activities in basal ganglia output nuclei, normal and MPTP-treated macaques underwent unitary recordings in the GPi. In agreement with previous reports, a significant increase (52%, MW; p<0.01) in the mean firing rate of GPi neurons was found in drug naive untreated MPTP macaques compared to controls (Fig. 4.a). Interestingly, striatal Lenti-TH-AAADC-CH1 administration significantly reduced the abnormal high firing rate and restored the firing rate of GPi neurons to normal (unlesioned) levels (Fig. 4.a).

The pattern of neuronal firing in the GPi is also important in the pathophysiology of PD, and so the burst activity of recorded GPi neurons was also analyzed. Pattern analysis revealed that the proportion of spikes per burst and the number of burst events significantly increased in MPTP primates (15.9 % and 9.7 events/cell/min respectively) compared to controls animals (3.8 % and 1.7 events/cell/min respectively; MW, p<0.05). Treatment with Lenti-TH-AAADC-CH1 significantly decreased the proportion of spikes per burst and the number of burst events in GPi neurons to levels that were very similar to those observed in normal unlesioned animals (5.3 % and 1.6 events/cell/min respectively; p<0.05) (Fig. 4.a).

Neuronal hyperactivity in the subthalamic nucleus (STN) is another key pathophysiological feature of PD, and its electrical neuromodulation has been therapeutically successful both in MPTP macaques and PD patients. Metabolic studies of basal ganglia both in PD patients and primate models of nigrostriatal degeneration have demonstrated alterations in the cortico-basal loops. Using [¹⁴C] 2-deoxyglucose (2-DG) (150 µm spatial resolution) functional imaging, it was found that the STN in an MPTP control macaque showed an increase in metabolic activity compared to a normal macaque (Fig. 4.b). At 36 weeks post treatment with Lenti-TH-AAADC-CH1, the metabolic activity of both the right and left STN were normalized and closely resembled those of an unlesioned animal (Fig. 4.b).

### Example 4 - Dyskinesia studies

A major challenge in PD is to restore dopaminergic function without inducing any dyskinetic movement. Because striatal histological abnormalities can induce dyskinesia, the morphological alterations following gene transfer were investigated. Whereas all needle tracks were located in the putamen (Fig. 14), neuronal markers (Neu-N) exhibited no abnormalities in Lenti-TH-AADC-CH1-injected animal. A slight increase in GFAP and CD68 immunoreactivities were observed in both Lenti-LacZ and Lenti-TH-AADC-CH1 animals. However this was restricted to the region surrounding the needle track (Fig. 15). A neuroimaging study was performed using T2*-weighted MRI, a sensitive method for the detection of local brain pathology. It was found that the putamen and the remaining brain areas were free from any abnormal T2* signal (Fig. 16).

One critical issue for the clinical application of Lenti-TH-AADC-CH1 is the potential of the vector to induce dyskinesias in PD patients. Evaluation of dyskinesias was performed in the efficacy studies describe above using a novel method for dyskinesia quantification based on video dyskinesia analysis (VDA) that assesses the whole range of dyskinetic movement continuously during the observed video sequence, using a standardized protocol. Lenti-TH-AADC-CH1 did not induce any dyskinetic movements in MPTP primates up to 30 months, in contrast to oral L-Dopa intake in control MPTP animals (n=5) (Fig. 5). In addition, MPTP primates who received Lenti-TH-AADC-CH1 displayed a reduction in their Off state dystonia as compared to MPTP primates and MPTP-Lenti-lacZ primates (MW, p< 0.05) (Fig.5).

To mimic clinical conditions, these drug-naive animals were further challenged with acute systemic administration of L-Dopa then with a pro-dyskinetic short-acting D1/D2 dopaminergic agonist (apomorphine). Oral L-Dopa intake and apomorphine injection both improved the locomotor activity of MPTP-*long term* and MPTP-LacZ primates to levels similar to that of normal primates (MW, p<0.05) (Fig. 17), whereas the locomotor activity of drug naive normal and MPTP-Lenti-TH-AADC-CH1 primates were unchanged. Furthermore, normal and MPTP-Lenti-TH-AADC-CH1 animals were free from dyskinesia following both a standard dose of L-Dopa and apomorphine, whereas MPTP-*long term* and MPTP-Lenti-LacZ animals displayed debilitating choreiform and dystonic movements (MW, P<0.05) (Fig. 5).

Having demonstrated the capability of dopamine gene therapy to prevent dyskinesia, the approach was subsequently tested in a primate model of L-Dopa induced dyskinesia (LID) to see if ProSavin treatment could reverse dyskinesias that were already established in the animal model.

A group of six MPTP-treated primates were treated with repeated, daily oral L-Dopa, from 20mg/kg/d to 100mg/kg/d, adjusted for each individual, until animals developed sustained and severe LID (LID-MPTP animals) (n=6). The animals then received bilateral injections into the motor striatum (as performed in the MPTP-treated drug-naïve primates) of either Lenti-TH-AADC-CH1 (n=3) or Lenti-lacZ (n=3). To closely mimic the clinical trial situation in LID PD patients, we then adjusted the daily dose of L-Dopa treatment for each individual animal (in the MPTP-LacZ and MPTP-Lenti-TH-AADC-CH1 group) to maintain the daily locomotor activity at its pre-MPTP-lesioning value. Thus changes in the dose of the daily L-Dopa treatment were based only on the "OFF" drug motor state (as evaluated with VMA). This approach is currently used to find optimal L-Dopa doses in patients implanted with deep brain stimulation electrodes (Bejjani et al (2000) Ann Neurol 47:655-658). Following striatal injections of Lenti-TH-AADC-CH1, LID-MPTP animals progressively recovered from their parkinsonism in the OFF L-Dopa state. Accordingly the treatment management protocol led to a progressive decrease of average L-Dopa intake in the LID-MPTP-Lenti-TH-AADC-CH1 animals from 70mg/kg/day to 30mg/kg/day at 6 months after vector injection, whereas daily L-Dopa treatment was stable at 67 mg/kg/day in LID-MPTP-Lenti-LacZ since no behavioural recovery was observed.

In the ON L-Dopa state (periodic challenge with a set dose of 40mg/kg of L-DOPA), the level of dyskinesia in Lenti-TH-AADC-CH1 treated animals was decreased and reached less than 25% of their initial levels (Fig. 18). On the contrary, the LID-MPTP primate that received Lenti-LacZ remained significantly impaired and continued to show a constant level of dyskinesia in response to L-DOPA challenges throughout the study (Fig. 18).

These results indicate that treatment with Lenti-TH-AADC-CH1 has the potential to not only provide therapeutic efficacy in a PD patient that has already developed dyskinesias from long term L-Dopa treatment but also to reverse the physiological mechanism of dyskinesias and thus prevent subsequent occurrences following L-Dopa therapy.

### Discussion

A principle mechanistic hypothesis for the onset of dyskinesia in PD is that it is a consequence of intermittent pulsatile dopaminergic stimulation of post-synaptic receptors in the striatum combined with extensive degeneration of the dopaminergic neurons of the nigral striatal tract. Here it is demonstrated that lentiviral continuous delivery of dopamine into the striatum mediated long term correction of motor deficits (up to 30 months) and, in contrast to repeated L-DOPA treatment, did not result in the occurrence of dyskinesias. This indicates that it is not the extent of nigral degeneration *per se* that causes drug induced-dyskinesia, but rather the absence of a constant dopaminergic tone in the striatum.

The most likely explanation for the observed results is that the gene therapy has restored sufficient dopamine to maintain the dopaminergic tone in the striatum and that this restores normal functioning of the basal ganglion networks as demonstrated by normalization of GPi neuronal activity and STN metabolism. No other neurological or anatomical changes were found that could support an alternative hypothesis. The therapeutic effects described herein have been achieved with modest and localized gene transfer generating sub-physiological levels of dopamine. Assuming that the snapshot obtained with micro-dialysis reflects the active dopamine concentration at the cell surface/extracellular space, our gene therapy procedure restores the dopamine concentration in the putamen to about 50% of normal levels. How could 50% dopamine replacement account for dramatic behavioural correction? This result is consistent with the observation in human Parkinson's disease that motor symptoms are observed when greater than 60% of the dopaminergic neurons have degenerated. Therefore a modest replacement of dopamine in the striatum would be expected to provide therapeutic benefit. In addition, the behavioural improvement observed in this study may also reflect a combination of the sub-normal lentiviral dopamine production and the reduced levels of the dopamine transporter (DAT) in the Parkinsonian striatum due to the decrease in the number of presynaptic synaptic dopaminergic terminals. The DAT is believed to control spatial and temporal activity of release dopamine and lower levels may increase the activity and/or distribution of dopamine released from the Lenti-TH-AADC-CH1 transduced striatal neurons (Giros et al (1996) Nature 379:606-612).

The current validated surgical treatment for PD involves electrical stimulation of the STN or GPi and this prevents L-Dopa induced motor complications by restoring normal electrical activity within these nuclei. It is possible that gene therapy may provide these therapeutic benefits but without the neuropsychological side effects observed by unwanted electrical stimulation of non motor regions of the STN.

An alternative approach for achieving continuous dopamine production in the striatum is to use cell grafting of foetal tissue or stem cells. However, graft-induced dyskinesias and the question of the development of PD pathology in transplanted cells have not yet been resolved. The data presented here indicate that the medical challenge of developing PD therapies capable of local and continuous stimulation of dopamine receptors has now been achieved.

### Methods summary

### Lentiviral vector technology

A tricistronic lentiviral vector was designed that encodes the genes for TH, AADC and CH1 (Lenti-TH-AADC-CH1). To improve vector-mediated dopamine production, a number of changes were made to the original EIAV vector genome that expressed the tricistronic cassette called pONY8.1TSIN (Azzouz et al (2002) J. Neurosci. 22:10301-10312). These changes led to at least a 2 log increase in dopamine production per integrated genome as assessed *in vitro* after transduction of human HEK293T cells (Fig. 6).

### Local dopamine depletion in animal models

To model advanced PD in non-human primates, the selective neurotoxin MPTP was systemically administered to adult *Macaca fascicularis* until they reached a severe and stable bilateral Parkinsonian syndrome, including akinesia, flexed posture, balance impairment and tremor. Before MPTP treatment, all primates scored 0 on the clinical rating scale (CRS). After MPTP, but before any lentiviral injection, macaques displayed a significant increase in the CRS approaching the maximal disability score (max=14) compared to the control pre-MPTP state (Normal state; Fig. 1.a). The severity of motor impairment was further quantified using quantitative video-movement analysis, and it was found that, compared to the Normal state, MPTP macaques displayed a marked akinesia (travelled distance=3.7% of Normal state; Fig. 1.b) and posture impairment (rearing activity=5% of Normal state; Fig. S2). The severity of MPTP induced Parkinsonism was stable over the course of the entire experiment in control MPTP animals (Figs. 1 and 7). Neuropathological analysis demonstrated selective nigro-striatal degeneration, including both structural and functional loss in the substantia nigra pars compacta (Fig. 8) and a dramatic decrease of TH and AADC immunoreactive fibers in the striatum (Fig. 2). Striatal denervation as assessed by TH (Fig. 2) and dopamine transporter (DAT) immmunoreactivity (Fig. 9) demonstrated an heterogeneous pattern of degeneration resembling that observed in PD, the putamen being more affected than the caudate nucleus, and the dorsolateral part of the putamen ('motor' putamen) more affected than its ventral part ('cognitive' putamen).

The construction and production of lentiviral vectors was performed by Oxford BioMedica. Lentiviral vectors were derived from EIAV, and encoded for either TH-AADC-CH1 in the same polycistronic vector (Lenti-TH-AADC-CH1), or for lacZ as a control (Lenti-lacZ). Twenty-six adult male Macaca fascicularis were used. The synthetic agent 1-methyl-4-phenyl-1,2,3,6-tetrahydropyridine (MPTP) is a neurotoxin that is *transformed in vivo* into MPP+, which has both high-affinity and high-toxicity for dopaminergic neurons. The MPTP macaque is considered as the most predictable among preclinical experimental models of PD. A subchronic protocol for MPTP intoxication was used(0.2 mg/kg/day). Objective behavioural analysis was used to determine when MPTP treatment should be halted. As soon as the primates had reached the behavioural criteria that corresponded to advanced PD model, MPTP treatment was stopped. Special attention was made to feed and nurse the animals, especially following MPTP lesioning. Lentiviral vectors were injected into the motor putamen of MPTP macaques. All surgical procedures used individual MRI based stereotaxy. Behavioural analysis used both automated quantitative video approaches, and qualitative clinical evaluation made in strict blind conditions. Immunohistochemistry and stereology were performed using standard techniques. Dopamine production was assessed by HPLC analysis of *in vivo* microdialysis samples and post-mortem brain tissue. L-Dopa was administrated orally, except during microdialysis experiments where it was injected i.m. Apomorphine was injected i.m. Single cell electrophysiology recording was performed using standard techniques. Functional imaging was performed by assessment of local cerebral glucose utilization using [¹⁴C]-2-Deoxyglucose (2-DG)^{43,44}. Data were analysed with Kruskal-Wallis (KW) or Friedman test (the non-parametric equivalent of the repeated measures ANOVA) and then, with Mann-Whitney (MW) post-hoc test at the individual time-points, corrected for multiple comparisons.

**Animals.** All animal studies were conducted in accordance with the European convention for animal care (86-406) and the NIH's Guide for the Care and Use of Laboratory Animals. Twenty-six adult male *Macaca fascicularis,* weighing 5-7 kg, were included in this study. Animals were housed individually with a 12/12h light/dark cycle. Following lentiviral vector injections, experiments were performed using level III Biosafety procedures (BSL3).

**Experimental Design.** Neurotoxin MPTP was first administrated to 23 macaques until they developed severe Parkinsonism, these were then divided into experimental groups according to the following experiments:
1. In the first experiment the long term effects of local and continuous dopaminergic production mediated by Lenti-TH-AADC-CH1 were studied. Eighteen MPTP non-human primates were randomized and assigned to 3 groups: striatal injection of Lenti-TH-AADC-CH1 (MPTP- Lenti-TH-AADC-CH1 group, n=6 until 8^{th} week then n=3 until 9^{th} Month), striatal injection of Lenti-LacZ (MPTP- Lenti-LacZ, n=6 until 8^{th} week then n=3 until 9^{th} Month) or no treatment (MPTP-*long term* group, n=6 until 8^{th} week then n=3 until 9^{th} Month). The animals were then observed from 2 up to 30 months. A subset of these primates were included for histology, microdialysis, electrophysiology, and metabolism studies.
2. In the second experiment the incidence of dyskinesia in the Parkinsonian primates who received dopamine gene therapy was investigated (MPTP Lenti-TH-AADC-CH1, n=6 until 8^{th} week then n=3 until 9^{th} Month). Dyskinesia incidence was compared to that observed in Parkinsonian primates who received oral pharmacological dopaminergic treatment. For the latter group, five MPTP macaques received daily oral L-Dopa for 12 weeks (MPTP-L-Dopa group, n=5).
3. In the third experiment the potential of dyskinesia induction in MPTP macaques was studied by challenging them with a short acting D1/D2 dopaminergic agonist, apomorphine, then with an acute oral dose of L-Dopa. Animals from the MPTP- Lenti-TH-AADC-CH1 group(n=3), MPTP- Lenti-LacZ group (n=3), MPTP-long term group (n=3), or normal unlesioned macaques (n=3) were challenged with apomorphine and with L-Dopa, 32 weeks after cessation of MPTP administration, and 24 weeks after gene transfer (for MPTP-Lenti-TH-AADC-CH1 *long term* animals), and the number of dyskinesias they expressed was counted with a specific software (The Observer®, Noldus).
4. In the fourth experiment the potential of continuous dopaminergic stimulation mediated by Lenti-TH-AADC-CH1 to reduce LID expression in primed dyskinetic MPTP animals was studied. A subset of 3 MPTP primates rendered dyskinetic (maximum score on dyskinesia index) by daily oral L-Dopa intakes received motor putamen injection of Lenti-TH-AADC-CH1 (n=2) or Lenti-LacZ (n=1). Animals were then challenged with L-Dopa and the number of dyskinesias was assessed with a specific software (The Observer®, Noldus)

For all MPTP-treated animals, the complete study involved daily clinical assessment, plus a series of behavioural analyses. One additional normal, non MPTP-treated, animal was included for immunohistochemistry and biochemistry studies. Two MPTP-treated and two unlesioned animals were included in the imaging and electrophysiological studies. Autoradiography imaging studies were performed in the final stage of the experiments, one month after electrophysiological recordings in the GPi.

**Viral production.** To improve vector-mediated dopamine production, a number of changes were made to the original EIAV vector genome that expressed the tricistronic cassette called pONY8.1TSIN (Fig. 9). This construct contains the catalytic isoforms of the following three genes: human aromatic L-amino acid decarboxylase (AADC, accession no M76180), human tyrosine hydroxylase (hTH-2, accession no X05290) and human GTP cyclohydrolase 1 (CH1, accession no U19523). All three of these genes were tagged using different N-terminal peptide tags. The new construct, pONY8.9.4TY, was generated by codon optimizing the sequences for TH, AADC and CH1 and removing all N-terminal tags. This was carried out by Operon (now Qiagen, Valencia, CA 91355). The order of the genes in the tricistronic cassette was also changed such that the TH open reading frame is first followed by AADC and CH1 (Lenti-TH-AADC-CH1). Both promoter and IRES sequences used were kept the same as in pONY8.1TSIN (Fig. 9). In addition the backbone was changed in the following way: all the ATG potential start codons in gag were changed to ATTG, a Neo expression cassette was inserted downstream of gag and the WPRE was inserted at the 3' end of the Tricistronic cassette to enhance expression. Figure 19 shows sequence of pONY8.9.4TY. These changes led to at least a 2 log increase in dopamine production per integrated genome as *assessed in vitro* after transduction of human HEK293T cells (Fig. 9). A LacZ encoding version of this vector (pONY8.9NCZ) was used as a control. pONY8.9NCZ (Lenti-lacZ) contains the LacZ ORF instead of the Tricistronic cassette.

Generation of Viral Vector. HEK293T cells were seeded in DMEM-HEPES with 10% (v/v) FCS at a density of 4.4 x 10⁴cm⁻² in a 10 layer cell factory. The next day cells were transfected with the pESYNGP (an EIAV codon optimized gag/pol expression construct), pRV67 (a VSV-G envelope expression plasmid) and EIAV-lacZ (an EIAV vector genome expressing LacZ under the control of the human cytomegalovirus immediate-early enhancer/promoter, or Lenti-TH-AADC-CH1 using Fugene-6 (Roche). Sixteen hours after transfection the cells were treated with 10mM Sodium Butyrate for 6 hours and then the culture medium was replaced with butyrate-deficient medium. At 40 hours post transfection the culture medium was **collected**, centrifuged at 1000x g for 5 min and filtered through a 0.45µm filter unit. The vector was concentrated by low speed centrifugation (6000x g for 16h at 4°C) followed by ultracentrifugation (50000x g, for 90 minutes at 4°C). The vector was resuspended in TSSM buffer consisting of sodium chloride (100mM), Tris, pH 7.3 (20mM), sucrose (10mg.ml) and mannitol (10mg.ml), aliquoted and stored at - 80°C. The vector titre was obtained by carrying out a DNA integration assay. Briefly this involves transducing HEK293T cells with the viral vector and passaging the cells for approximately 10 days. The titre of Lenti-lacZ was 3.5 x 10⁹ TU/ml and Lenti-TH-AADC-CH1 1.0-2.7 x 10⁸ TU/ml.

Behaviour. All animals were assessed daily for their general clinical condition with special attention paid to the nutritional status. At the same time, the general neurological state of each macaque was assessed both pre- and post-lentiviral injections. To objectively quantify our neurological observations, animals were video-taped for 30 minutes before MPTP lesioning and at regular intervals following viral injections. The videos were then analyzed off-line by an examiner blinded to the experimental conditions. A clinical rating-scale was adapted from the Papa and Chase MPTP primate Parkinsonian scale (posture 0-2, gait 0-2, tremor 0-2, general mobility 0-4, hand movements 0-2, climbing 0-4; a score of 0 corresponds to a normal monkey). The quantitative analysis of dyskinesia was performed using a motion counting software (The Observer 7, Noldus, Wageningen, The Netherlands) that allowed to count predefined movements (superior and inferior members, trunk, face and neck chorea and dystonia for dyskinetic movements) during the video recording period. Video-movement analysis was performed using a motion tracking software (Ethovision 3, Noldus, Wageningen, The Netherlands) that allowed an objective measurement of total distance moved (travelled distance, cm), maximum velocity (maximal velocity, cm/sec) and rearing behaviour frequency (rearing, number of events) during the video-recording period.

MPTP Lesion. All primates received a daily intramuscular dose of 0.2 mg/kg of MPTP (Sigma Aldrich, St Louis, Missouri) until they reached a severe stable bilateral Parkinsonian syndrome. MPTP administration was halted when animals reached an increase of the clinical rating scale to ≥ 10 and a decrease of travelled distance ≤ 500 cm/30min, maximal velocity ≤ 5 cm/sec/30min, rearing ≤ 5/30min, as assessed by video-recording session performed 1 week after last MPTP injection. The stability of Parkinsonian syndrome was then checked, using same behavioural criteria, during the 8 weeks following the last MPTP injection.

Viral injection procedure. Under generalized anaesthesia with a mixture of Ketamine and Xylazine (15 mg/kg + 1.5 mg/kg, every hour), nine Parkinsonian MPTP macaques received five stereotaxic injections of Lenti-lacZ or Lenti-TH-AADC-CH1 bilaterally (10 µl/injection i.e. 50µl/putamen and a total of 100µl per animal) into the commissural (10 µl) and post-commissural putamen (10 µl x 4) under sterile conditions. Target coordinates were based upon MRI guidance (1.5-T MR magnet, General Electric medical system, Waukesha, Wisconsin) using neuronavigation methods. The first injection was aimed at the commissural level of the putamen followed by a group of 2 injections (second and third injection, 1 mm apart) 2 mm caudal from the anterior commissure and by a second group of 2 injections (forth and fifth injection, 1 mm apart) 5 mm caudal from the anterior commissure. All injections were placed in the dorsolateral part of the putamen. Lentiviral vector was injected manually in each of the 10 stereotactical tracks through a 10-µl Hamilton syringe at a rate of 1 µl/min. The needle was left *in situ* for an additional 2 min. All needle tracks were bilaterally located in the putamen as observed by Nissl staining and immunohistochemical studies (Fig. 14).

**Immunohistochemistry.** Eight weeks after lentiviral vector injections, six macaques were deeply anesthetized with ketamine (15 mg/kg), euthanized by an overdose of pentobarbital (100 mg/kg, intravenously; Sanofi, France) and perfused transcardially with cold saline. Brains were removed immediately, hemisected by a midline sagittal cut and slabbed on a monkey brain slicer. Slabs through the right putamen were punched for HPLC studies, using cylindric brain punchers (internal diameter 1.5mm). Length of punches was approximately 1mm. The tissue slabs were then immersed in a cold 4% paraformaldehyde fixative solution for 6 days, washed in a series of cold graded sucrose solutions for 4 days and sectioned in a coronal plane on a freezing microtome (sections 40 µm in thickness). Sections for immunohistochemical labeling were first incubated for 48h at room temperature or 72 h at 4°C in phosphate buffer saline containing 0.5% triton-X100, 2% bovine serum albumin, 3.5% normal serum and the appropriate dilution of the first antibody: anti-TH, 1:1000 dilution (Institut Jacques Boy, Reims, France); anti-AADC, 1:250 dilution (Chemicon, CA); anti-dopamine, 1:1000 dilution (AbCam, Cambridge, UK), anti-CHI, 1:3000 (a kind gift from Ernst Werner, University of Innsbruck); anti-β-Gal, dilution 1:2000 (Chemicon, CA), anti-NeuN, dilution 1:5000 (Chemicon, CA), anti-DAT, dilution 1:7500 (Chemicon, CA), anti-GFAP, dilution 1:30000 (DakoCytomation, Glostrup, Denmark), anti-CD68, dilution 1:100 (DakoCytomation, Glostrup, Denmark). After incubation in the primary antiserum, sections were processed with the avidin-biotin peroxidase method. The TH transgene used in the present lentiviral vector is a truncated form (trunc-TH2) of the human TH isoform 2 (hTH2). To study the expression of striatal transgenic TH, a TH antibody that recognizes both N-terminal regulatory and C-terminal catalytic units of hTH2 was used.

**Double Labelling Immunofluorescence Procedure.** To identify the cell types transduced with EIAV vectors, an indirect immunofluorescence double-label technique was employed. An indirect immunofluorescence double-label technique was employed to label βGal-positive cells in the striatum of EIAV-lacZ injected animals with a neuronal (NeuN) and glial (GFAP) markers. For each experiment, background staining was inhibited with a 1-h incubation in a blocking solution (4.5% normal goat serum and 0.2% Triton X-100 in PBS, pH 7.4) at room temperature. Sections were then incubated in primary rabbit polyclonal antibody to βGal (AbCam, 1:1000) overnight at room temperature. After washes, the sections were incubated in the secondary goat anti-rabbit IgG coupled to the fluorescent marker Alexa 488 (1:200) for 1 h. After washes, sections were incubated in one of the following the primary antibodies: mouse monoclonal anti-NeuN (Chemicon; 1:1000), mouse monoclonal anti-GFAP (Sigma; 1:3000), overnight at RT. After incubation in the secondary antibody (biotinylated goat anti-mouse IgG 1:200) for 1 h at room temperature, the sections were placed in fluorolink Cy 3-labeled streptavidin (1:1000) for 1 h at room temperature. All fluorescence images were analyzed with the Zeiss Confocal Fluoroview microscope equipped with argon and He-Ne lasers.

**Stereological Analysis.** Stereological analysis was used for cell counts. To evaluate the total number of EIAV-positive cells within the striatum, i.e. number of transduced cells, alternate sections were stained for β-galactosidase immunoreactivity (βGal-ir) and positive cells counted throughout the entire striatum of Lenti-lacZ injected animals. To evaluate the total number of remaining dopaminergic cells within the *substantia nigra*, alternate sections stained for tyrosine hydroxylase immunoreactivity (TH-ir cells) were counted throughout the entire SNpc of unlesioned normal controls, MPTP-Lenti-lacZ and MPTP-Lenti-TH-AADC-CH1 injected animals. Stereological count of cells was processed using Olympus stereology software C.A.S.T.-Grid (Olympus Denmark, Albertslund, Denmark) and a computer-assisted image analysis system (Olympus Pentium II) linked to an Olympus Provis microscope (Olympus France, Rungis, France) equipped with a video camera (HAD Power 3CCD, Sony) and a computer-controlled motorized stage. Stereological analyses used the optical fractionator procedure, a design-based stereological method for estimating total number of structures in a known fraction of a defined reference space without being affected by tissue shrinkage.

For the striatal EIAV-positive cell counting, a coefficient of error of <0.10 due to the estimation was accepted. For the SNpc TH-ir cells, a higher coefficient of error (<0.35) was accepted. This was related to the dramatic decrease in the number of TH-ir cells after the MPTP intoxication, leaving a very few objects to count.

**L-Dopa administration.** The animals in the L-Dopa group were treated chronically with an average daily oral dose of 20 mg/kg L-Dopa and benserazide (at a 4:1 ratio, Modopar dispersible®, Roche, France) (termed "L-Dopa" thereafter). During the L-Dopa challenge of MPTP, Lenti-LacZ and Lenti-TH-AADC-CH1 non-primed primates, animals received a single oral dose of 20mg/kg of L-Dopa and benserazide (at a 4:1 ratio, Modopar dispersible®, Roche, France). During the microdialysis experiment, primates were challenged with a single dose of 40mg/kg i.m. of L-Dopa and benserazide (at a 4:1 ratio, methyl-esther-L-Dopa, Sigma Aldrich, St Louis, Missouri).

**Short acting D1/D2 Agonist administration.** Apomorphine (Aguettant, Lyon, France), a short acting non selective D1/D2 agonist, was administered systemically at a dose (0.1 mg/kg i.m.) known to induce dyskinesia in MPTP-treated macaques.

**Microdialysis.** Primates were anesthetized with Ketamine and Xylazine (15 mg/kg + 1.5 mg/kg, every hour) and placed in a stereotaxic frame. The body temperature was stabilized at 37°C throughout the experiment with a thermostatic blanket. The microdialysis probes (CMA/12, membrane length 5 mm, cut-off 20 kDa; CMA Microdialysis, North Chelmsford, MA) were implanted bilaterally in the striatum. Microdialysis probes were placed into the post-commissural putamen of four normal unlesioned animals, four MPTP animals, three MPTP-Lenti-LacZ animals, and two MPTP-Lenti-TH-AADC-CH1 animals. Probes were perfused with aCSF (in mM: 147 NaCl, 2.7 KCl, 1.2 CaCl2, and 0.85 MgCl2) at a rate of 2 µl/min. Microdialysates were collected every 15 min into a refrigerated fraction collector and frozen at -80°C until analysis. Following implantation of each probe into the primate brain, microdialysis samples were taken over a 2 hour stabilisation period allowing recovery from any transient increase in neurotransmitter release due to procedural trauma. Baseline samples were then taken over the next hour. Then, following collection of baseline samples, animals were subjected to an acute Dopamine (Dopamine 40 mg/kg i.m.) or L-Dopa challenge (L-Dopa methyl ester, 40mg/kg i.m.) and additional microdialysis samples were taken continuously over a 2 hour period. By the end of the microdialysis session, additional control samples were generated by performing microdialysis for 30 minutes in a solution of known dopamine concentration (1µM) following removal of the probe from the putamen. This allows for the calculation of the efficiency of each probe and will enable estimation of the actual dopamine concentration in the putamen. After each experiment, the location of probes was checked using T2*-weighted MRI.

**Measurement of Dopamine *post*-*mortem*: whole tissue dopamine levels [DA]_{wt}.** High-performance liquid chromatography (HPLC) with electrochemical detection was used to measure striatal levels of catecholamines in brain punches and microdialysis samples. Briefly, brain punches were homogenized in homogenization buffer (1.2mM HEPES, 1% Triton X-100, 10% glycerol supplemented with protease inhibitors, pH 7.2). Catecholamines were extracted by mixing the tissue homogenates with one tenth of a volume of extraction buffer (0.4M perchloric acid, 0.1mM EDTA pH8.0). The supernatants were then centrifuged and filtered. Microdoalysis samples were treated with one sixth of a volume of 0.2M perchloric acid. The supernatants or microdialysis samples were applied to an HPLC system (Agilent 1100) equipped with an ESA Coulochem II electrochemical detector (ESA Analytical). Catecholamines from brain homogenates were separated using a HR-80 column (ESA Analytical) and Cat-A-Phase mobile phase (ESA Analytical) at a flow rate of 1.5 ml/min and then detected electrochemically.

**Measurement of Dopamine *in vivo* : extracellular dopamine levels [DA]_{ec}**. The sensitivity of the HPLC system was validated by running catecholamine standards through the HPLC and detection system and evaluation of output traces. Standard solutions containing L-Dopa, DOPAC, HVA and dopamine in the range of 10-1000 pg/ml were made up in a solution of 1:5 artificial CSF in standard diluent to ensure the same background signal as the microdialysis samples. One hundred microlitres of each standard solution (0.5-100pg of each catecholamine) was run through the HPLC system and the output for each metabolite analysed. Standard curves for each metabolite were generated from these data and the concentration of catecholamines in microdialysis samples was calculated from these curves.

Microdialysis samples were thawed and 5µl of 0.2M perchloric acid added to each sample prior to HPLC analysis. Samples were diluted 1 in 5 in standard diluent (ESA) and 100µl injected for each analysis. Further dilution (1/10) was required for analysis of L-Dopa and HVA levels using the remaining sample. Samples were run on an Agilent 1100 HPLC machine and separated using a MD-150 column (ESA Analytical) and MD-TM mobile phase (ESA Analytical) at a flow rate of 0.6ml/min and then detected electrochemically using a Coulchem II electrochemical detector (ESA). To improve efficiency of dopamine detection a specialised Microdialysis cell (5014B, ESA) was used in conjunction with the detector to allow detection of low levels (<1pg) of dopamine.

Electrophysiology. Under constantly monitored ketamine/xylazine anesthesia (15 mg/1.5 mg/kg), single-unit activities were recorded only during a time period when stable heart rate, respiratory frequency, and CO2 expiratory flow were observed. A glass-coated tungsten microelectrode was stereotactically implanted under MRI guidance into the internal Globus Pallidus. Recording locations were verified by histological reconstruction of the electrode tracks. Signal was amplified and bandpass filtered (300-5000 Hz) using Leadpoint (Medtronic, Minneapolis, MN). Single-cell action potentials were first threshold or template extracted, and only well-isolated units were selected for further analysis. Twenty second spike trains were recorded and stored for offline analysis. Neuronal activity was extracted using a threshold or template -matching algorithm (Dataview4.5; W.J. Heitler, University of St. Andrews, Scotland), and mean firing rates were calculated. Bursting discharge was quantified using the Poisson "surprise" method of burst detection with a Poisson surprise value of >10. The proportion of spikes in burst discharges compared with the total number of spikes sampled for each cell was determined.

**Local cerebral glucose utilization (LCGU) using [¹⁴C]-2-Deoxyglucose (2-DG).** LCGU was measured in macaques on the day they were sacrificed. Experiments were performed on animals anaesthetized with propofol. Body temperature was monitored rectally and maintained at 37°C using a thermostatically controlled heating pad. Polyethylene catheters were inserted into a femoral vein and artery for subsequent i.v. administration of 2-DG and sampling of arterial blood. The procedure was initiated by the infusion of an intravenous pulse of 100 µCi/kg 2-deoxy-D-[¹⁴C]glucose (PerkinElmer Life Sciences, Boston, MA; specific activity 50-55 mCi/mmol). Timed arterial blood samples (0.25, 0.5, 0.75, 1, 2, 5, 7.5, 10, 15, 25, 35, and 45 min) were drawn thereafter at a schedule sufficient to define the time course of the arterial 2-[¹⁴C]deoxyglucose and glucose concentrations. Arterial blood samples were centrifuged immediately. Plasma ¹⁴C concentrations were determined by liquid scintillation counting (Beckman Instruments, Fullerton, CA), and plasma glucose concentrations assessed using a glucometer (OneTouch® Ultra® Blood Glucose Monitoring System, Lifescan, Johnson&Johnson, Issy-les-Moulineaux, France). Forty-five min after tracer injection, the animals were sacrificed by an intravenous overdose of sodium pentobarbital (150 mg/kg i.v.). The brain was rapidly removed and frozen in isopentane (-45°C), and stored at -80°C for later autoradiography processing. Coronal brain sections (20 µm thick) covering the entire striatum, premotor and pre-frontal cortex (50 sections) were obtained at -20 °C with a cryomicrotome, mounted on superfrost slides, quickly dried and exposed onto an autoradiographic film (Kodak BioMax MR) for 7-10 days, with calibrated [¹⁴C]-standards (American Radiochemical Company, St Louis, MO, USA). The same brain sections were then stained with Cresyl Violet, and optical images from these sections were reconstructed in a 3D volume space. Autoradiograms were digitized and analyzed using a computer-based image analysis system (MCID Analysis, St. Catharines, Ontario, Canada). Then optical images from autoradiograms were co-aligned with the co-registered 3D anatomical space to provide anatomic and functional volumes. Optical densities determined in the STN on the autoradiograms (10-13 sections per STN) were converted to radioactivity and then to glucose consumption values using the [¹⁴C] standards and the modified operational equation of Sokoloff.

**Statistical Analysis.** Values are mean ± s.e.m. Data were analysed with Kruskal-Wallis (KW) or Friedman test (the non-parametric equivalent of the repeated measures ANOVA) and then, with Mann-Whitney (MW) post-hoc test at the individual time-points, corrected for multiple comparisons, using SPSS software (SPSS Inc., Chicago, IL).

## Claims

1. A lentiviral vector system which comprises a single vector comprising nucleic acid sequences which encode tyrosine hydroxylase (TH), amino acid DOPA decarboxylase (AADC) and GTP-cyclohydrolasel (CH1) for use in treating and/or preventing dyskinesias associated with oral L-dopa administration in a Parkinson's disease subject.

2. A vector system for use according to claim 1, which vector system has one or more of the following features:
(i) where the vector system comprises a tricistronic cassette, the order of the genes in the tricistronic cassette is TH-AADC-CH1
(ii) at least one of the ATG potential start codons in gag is changed to ATTG;
(iii) a Neo expression cassette is inserted downstream of gag; and
(iv) where the vector system comprises a tricistronic cassette, a WPRE is inserted at the 3' end of the Tricistronic cassette to enhance expression.

## Patentansprüche

1. Lentivirus-Vektorsystem, das einen Nukleinsäure-sequenzen, die Tyrosin-Hydroxylase (TH), Aminosäure-DOPA-Decarboxylase (AADC) und GTP-Cyclohydrolase 1 (CH1) codieren, umfassenden Einzelvektor umfasst, zur Verwendung bei der Behandlung und/oder Vorbeugung von mit oraler L-Dopa-Verabreichung assoziierten Dyskinesien bei einem Individuum mit Morbus Parkinson.

2. Vektorsystem zur Verwendung nach Anspruch 1, wobei das Vektorsystem eines oder mehrere der folgenden Merkmale aufweist:
(i) Umfasst das Vektorsystem eine tricistronische Kassette, so ist die Reihenfolge der Gene in der tricistronischen Kassette TH-AADC-CH1;
(ii) wenigstens eines der möglichen ATG-Startcodons in gag ist zu ATTG geändert;
(iii) eine Neo-Expressionskassette ist stromabwärts von gag inseriert; und
(iv) umfasst das Vektorsystem eine tricistronische Kassette, so ist zur Verbesserung der Expression ein WPRE am 3'-Ende der tricistronischen Kassette inseriert.

## Revendications

1. Système de vecteur lentiviral comprenant un vecteur unique comprenant des séquences d'acides nucléiques qui codent pour la tyrosine hydroxylase (TH), l'acide aminé DOPA décarboxylase (AADC) et la GTP-cyclohydrolase 1 (CH1) pour l'utilisation dans le traitement et/ou la prévention de la dyskinésie associée à l'administration de L-dopa par voie orale chez un patient atteint de la maladie de Parkinson.

2. Système de vecteur pour l'utilisation selon la revendication 1, lequel système de vecteur présente une ou plusieurs des caractéristiques suivantes :
(i) lorsque le système de vecteur comprend une cassette tricistronique, l'ordre des gènes dans la cassette tricistronique est TH-AADC-CH1
(ii) au moins l'un des codons d'initiation potentiels ATG dans gag est modifié en ATTG ;
(iii) une cassette d'expression de néo est insérée en aval de gag ; et
(iv) lorsque le système de vecteur comprend une cassette tricistronique, un WPRE est inséré à l'extrémité 3' de la cassette tricistronique pour améliorer l'expression.
